# EUROPEAN PATENT SPECIFICATION

(11) **EP 2 995 311 B1**
(45) Date of publication and mention of the grant of the patent: **26.05.2021**
(21) Application number: 14184632.9
(22) Date of filing: 12.09.2014
(51) Int. Cl.: A61K 31/7004, A61K 31/015, A61K 36/41, A61K 36/254, A61K 36/79, A61P 9/10

(54) **Compositions for the treatment of age related disorders**
Zusammensetzungen zur Behandlung altersbedingter Erkrankungen
Compositions pour le traitement de troubles liés à l'âge

(43) Date of publication of application: 16.03.2016
(73) Proprietor: SHI Research & Development AB, 412 62 Göteborg (SE)
(72) Inventor: WIKMAN, Karl Georg, 41262 Göteborg (SE); PANOSSIAN, Alexander, 31275 Vaxtorp (SE)
(74) Representative: Lang, Johannes

(56) References cited:
- US-A1- 2008 118 582
- US-A1- 2010 080 821
- ALEXANDER PANOSSIAN ET AL: "Synergy and Antagonism of Active Constituents of ADAPT-232 on Transcriptional Level of Metabolic Regulation of Isolated Neuroglial Cells", FRONTIERS IN NEUROSCIENCE, vol. 7, 1 January 2013 (2013-01-01), XP055171176, ISSN: 1662-4548, DOI: 10.3389/fnins.2013.00016
- A PANOSSIAN ET AL: "Molecular chaperons mediated pathways of stress protective and anti-aging effects of adaptogens", PLANTA MEDICA, vol. 75, no. 09, 1 July 2009 (2009-07-01), XP055171686, ISSN: 0032-0943, DOI: 10.1055/s-0029-1234262
- ASLANYAN G ET AL: "Double-blind, placebo-controlled, randomised study of single dose effects of ADAPT-232 on cognitive functions", PHYTOMEDICINE, GUSTAV FISCHER VERLAG, STUTTGART, DE, vol. 17, no. 7, 1 June 2010 (2010-06-01), pages 494-499, XP027012956, ISSN: 0944-7113 [retrieved on 2010-04-16]
- KORMOSH N ET AL: "Effect of a combinaiton of extract from several plants on cell-mediated and humoral immunity of patients with advanced ovarial cancer", PHYTOTHERAPY RESEARCH, JOHN WILEY & SONS LTD. CHICHESTER, GB, vol. 20, no. 5, 1 May 2006 (2006-05-01), pages 424-425, XP002637853, ISSN: 0951-418X, DOI: 10.1002/PTR.1889 [retrieved on 2006-04-18]
- PANOSSIAN A ET AL: "Adaptogens exert a stress-protective effect by modulation of expression of molecular chaperones", PHYTOMEDICINE, GUSTAV FISCHER VERLAG, STUTTGART, DE, vol. 16, no. 6-7, 1 June 2009 (2009-06-01) , pages 617-622, XP026102728, ISSN: 0944-7113, DOI: 10.1016/J.PHYMED.2008.12.003 [retrieved on 2009-02-01]
- DATABASE EMBASE [Online] ELSEVIER SCIENCE PUBLISHERS, AMSTERDAM, NL; 1959, POZZI P: "Pantothenic acid and experimental tumours. I. Effect of pantothenic acid on the growth and development of ehr lich adenocarcinoma in white mice (Neoplastic homologous graft)", XP002736379, Database accession no. EMB-0008383676 & ARCH. ITAL. PATOL. CLIN. TUMORI 1959, 1959,

## Description

### FIELD OF THE INVENTION

The present invention relates to compositions of parts of plants or extracts, their use as dietary food supplements or phytomedicine and a method of preparation of such compositions. Particularly the invention relates to compositions for treatment of age related disorders.

### BACKGROUND OF THE INVENTION

The use of plants for medicinal purposes, and the study of such use have a long history. Plants have been the basis for medical treatments through much of human history, and such traditional medicine is still widely practiced. Plants, parts of plants or extracts of plants are used to treat conditions and diseases. The therapeutic effect is based on the chemical compounds present in the plant. The combination of compounds present in the plant or extract defines the therapeutic effect. In phytomedicine, plant material is processed in a repeatable operation so that a discrete marker constituent is at a verified concentration. The plant material or extract is then considered standardized.

In phytomedicine often a combination of different plants, parts of plants or extracts is used.

An example of a phytomedicine containing different extracts is ADAPT-232. ADAPT-232 is a combination of natural compounds of plant origin, standardized for the content p-hydroxyphenethyl-gluco-pyranoside, and several lignans. It was used in Scandinavia since 1996 as a natural remedy. It has been shown to significantly improving attention and ability to concentrate expressed as a decrease in errors made and an increase in speed and accuracy of performance stressful cognitive tasks in various psychometric tests, both in healthy subject [Aslanyan et al., 2010], e.g. in cosmonauts [Bogatova et al., 1997; Panossian and Wagner, 2005] as well as in pneumonia patients in the course of antibiotic treatment [Narimanyan et al., 2005]. The mean duration of a standard antibiotic treatment significantly decreased in the group of patients receiving ADAPT-232 together with a standard treatment. Shorter therapy with antibiotics (5.67 days) compared with those receiving the placebo with a standard treatment (7.53 days) was required, that suggests beneficial health effect of ADAPT in the course of antibiotic treatment and recovery of patients [Narimanyan et al., 2005]. Panossian et al. (Frontiers in Neuroscience, 2013) describes effects of Adapt-232 on the transcriptional level of metabolic regulation of isolated neuroglial cells. Panossian et al. (Planta Medica, Congress Abstract 2014) describes anti-aging effects of adaptogens, e.g. Adapt-232.

Surprisingly it has been shown that 210 unique genes are deregulated due to synergistic interaction of constituents of combination of parts of plants or plant extracts belonging to *Crassulaceae, Araliaceae and Schisandraceae.* These genes are involved in the development of age related disorders.

Examples of age related disorders are:
- Deregulated level of apoptosis.
- Spontaneous occurrence of tumours.
- Dysfunction of hypothalamus-pituitary-adrenal system activity with influence on lipid and protein metabolism.
- Negative impact on blood fats like cholesterol, HDL cholesterol, triglycerides.
- Negative impact on carbohydrate metabolism like glucose.
- Chronic inflammation and atherosclerosis.
- Impaired health status and higher mortality

There are many aging associated diseases, which are developed because of age dependent increasing dysfunction and degeneration of vascular and immunocompetent cells. Examples of these diseases are cancer, gastrointestinal diseases, endocrine system disorders, inflammatory diseases, auditory diseases, cardiovascular diseases, immunological diseases, dermatological diseases and metabolic diseases. Surprisingly it has been shown that combinations of part of plants or extracts of plants containing a combination of phenethyl- and phenylpropenyl glycosides, lignans, flavolignans, epigallocatechingallates, mono-, sequi- and triterpene glycosides derived from plants belonging to *Crassulaceae, Araliaceae and Schisandraceae* are effective in the prophylaxis and treatment of age related disorders.

### BRIEF DESCRIPTION OF THE FIGURES

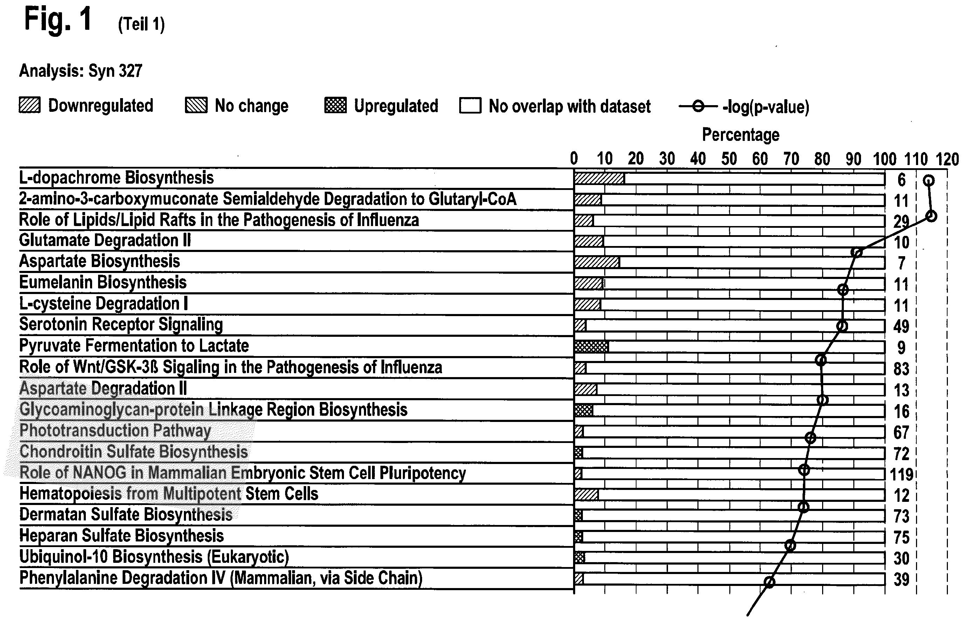

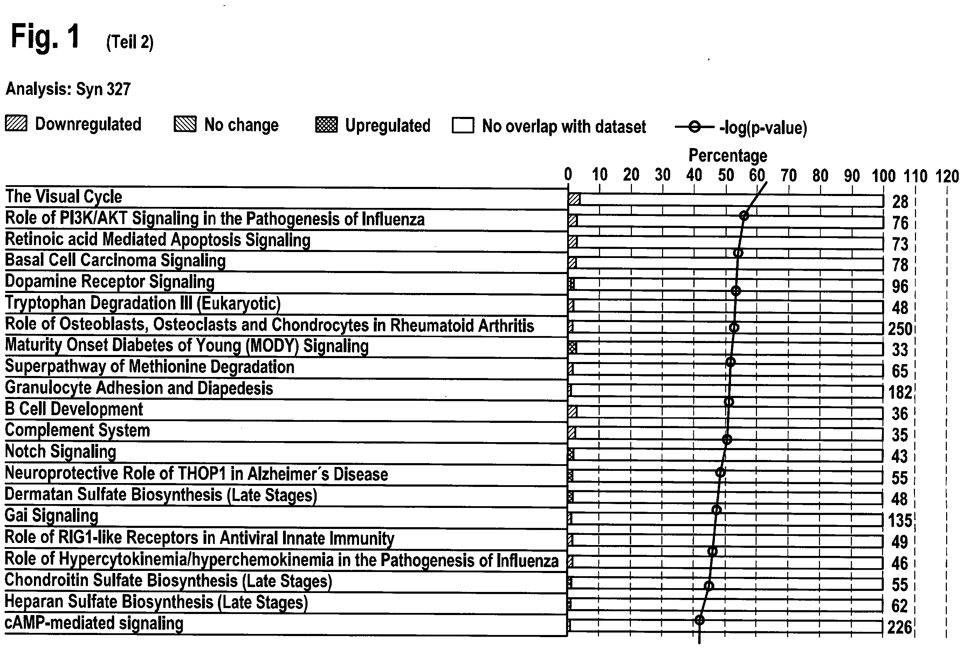

Figure 1 shows the main cellular functions most significantly affected by synergy derived effect of ADAPT-232.

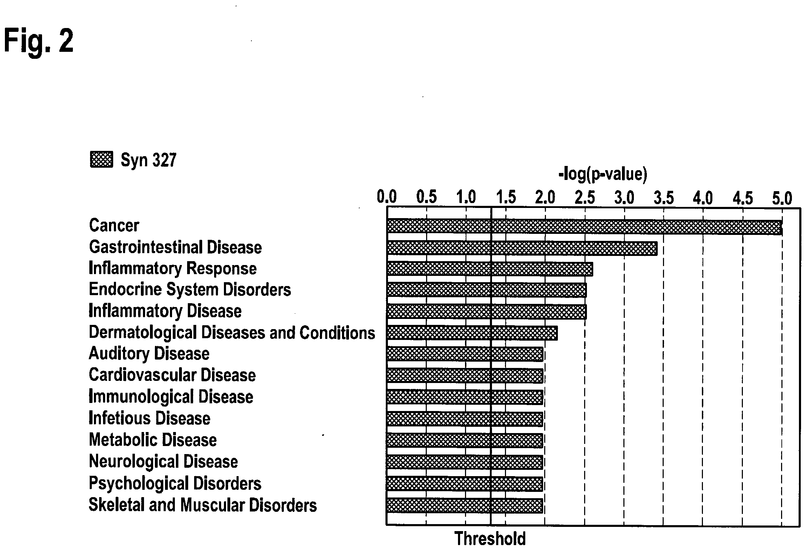

Figure 2 shows the main diseases most significantly affected by synergy derived effect of ADAPT-232.

### DETAILED DESCRIPTION OF THE INVENTION

The invention is set out in the appended set of claims. This invention is based on the unexpected finding that parts of plants or plant extracts of plants belonging to the family of Crassulaceae, Araliaceae and Schisandraceae comprising a combination of phenethyl- and phenylpropenyl glycosides, lignans, flavolignans, epigallocatechingallates, and mono-sequi-and triterpene glycosides are effective for prophylaxis, treatment and recovery of age related disorders.

Pantothenic acid or salts of pantothenic acid are added to the combination of extracts.

These parts of plants or plant extracts of plants belonging to the family of Crassulaceae, Araliaceae and Schisandraceae have a synergistic action on the expression of genes involved in development of age related disorders such as atherosclerosis, carcinogenesis, hypercholesterolemia, reduced physical endurance and liver detoxifying function, impaired protein synthesis, reduced activity of the hormonal system, and spontaneous promotion of tumours.

The invention is thus directed to compositions for the treatment or prevention of the prevention and treatment of age related diseases and conditions.

The surprising synergistic effect of these extracts was demonstrated in isolated neuroglia cells and the beneficial effect of ADAPT-232 in aging was demonstrated in experiments in rats. ADAPT-232 has a homeostatic and anti-aging action on the age-related deterioration of function of the innate defence, cardiovascular and carcinogenesis. Repeated administration of ADAPT-232 diminish or prevent a range of age-related disorders including development and progression of cardiac insufficiency and hypercholesterolemia, reduced physical endurance and impaired protein synthesis, reduced activity of the hormonal system and spontaneous promotion of tumours.

Examples of age related disorders are:
- Deregulated level of apoptosis.
- Spontaneous occurrence of tumours.
- Dysfunction of hypothalamus-pituitary-adrenal system activity with influence on lipid and protein metabolism.
- Negative impact on blood fats like cholesterol, HDL cholesterol, triglycerides.
- Impaired health status and higher mortality

For preventing ageing associated disorders the understanding of specific sets of genes involved in the ageing is important. Therefore the deregulation of the expression of specific set of genes, molecular networks and cellular intracellular signalling pathways by the compositions of the invention was investigated. Surprisingly it was established that the compositions of the inventions have a synergistic effect on the genes involved in development of age related disorders such as atherosclerosis and carcinogenesis.

The compositions of the invention decrease the cholesterol level in blood. Cholesterol is a lipid substance that is found in all body cells. It is located mainly in cell membranes, lipoproteins and metabolized into steroid hormones. The determination of serum cholesterol is one of the important tools in the diagnosis of atherosclerosis. High blood cholesterol is one of the major risk factors for heart disease. By the compositions of the invention the level in blood is reduced by more than 10%, preferably more than 20% and more preferably by more than 30% and even more preferably by more than 40%.

With respect to triglyceride the compositions of the invention stabilize the level of triglyceride.

The compositions of the invention also increase the level of albumin and protein in blood. In age related disorder the content of these compounds is often reduced. In a preferred embodiment the level is increased by more than 5% and in a more preferred embodiment by more than 10%.

The level of apoptosis is significantly influenced by the compositions of the invention. In a preferred embodiment the level of apoptosis is at least 10% less compared to placebo and more preferably at least 20% less and even more preferably at least 30% less compared to placebo.

The compositions of the invention are pharmaceutical compositions or dietary food products.

The invention relates to compositions of parts of plants or extracts of plants belonging to the families of *Crassulaceae, Araliaceae and Schisandraceae.*

Examples of plants from the plant family of *Crassulaceae* are Sedum rosea, Sedum maximum, Sedum auglicum, Sedum aruum, Sedum quadrifida, Sedum integrefolia, Sedum telephium, Sedum algida, Sedum crenulata, Sedum pinnatifida, Sedum hybridum, Sedum aizoon, Sedum purpureum, Sedum heterodonta, Sedum viridula, Sedum kirilowii, Sedum linearifolia, Sedum gelida, Sempervivum soboleferum. Especially suitable are the plants Sedum rosea and Sempervivum soboleferum. Examples of plants from the plant family of *Araliaceae* are Aralia elata, Aralia mandshurica, Eleutherococcus divaricatus, Eleutherococcus eleutheristylus, Eleutherococcus giraldii, Eleutherococcus nodiflorus, Eleutherococcus rehderianus, Eleutherococcus rufinervis, Eleutherococcus scandens, Eleutherococcus senticosus, Panax ginseng. Especially suitable are the plants Eleutherococcus senticosus and Aralia mandshurica.

Examples of plants from the plant family of *Schisandraceae* are Schisandra arisanensis, Schisandra bicolor, Schisandra chinensis, Schisandra tuberculata, Schisandra flaccidiramos, Schisandra glabra, Schisandra glaucescens, Schisandra henryi, Schisandra incarnate, Schisandra lancifolia, Schisandra micrantha, Schisandra neglecta , Schisandra plena, Schisandra propinqua and Schisandra tomentella. Especially suitable is the plant Schisandra chinensis.

Examples of the parts of the plants used are stems, stem barks, trunks, trunk barks, twigs, tubers, roots, toot barks, young shoots, seeds, rhizomes, flowers, fruits or leaves.

The combination of the invention comprises a combination of the chemical components phenethyl- and phenylpropenyl glycosides, lignans, flavolignans, epigallocatechingallates, mono-sequi-and triterpene glycosides.

A combination is described which contains one or more of the following compounds:
- (2R,3S,4S,5R,6R)-2-(hydroxymethyl)-6-[2-(4-hydroxyphenyl)ethoxy]oxane-3,4,5-triol (salidroside)
- 4-(2-hydroxyethyl)phenol (tyrosol)
- (2S,3R,4S,5S,6R)-2-[(E)-3-phenylprop-2-enoxy]-6-[[(2S,3R,4S,5S)-3,4,5-trihydroxyoxan-2-yl]oxymethyl]oxane-3,4,5-triol (rosavin)
- (2R,3S,4S,5R,6S)-2-(hydroxymethyl)-6-[4-(3-hydroxyprop-1-enyl)-2,6-dimethoxyphenoxy]oxane-3,4,5-triol (eleutheroside B)
- (2R,3S,4R,5R,6S)-2-[4-[6-[3,5-dimethoxy-4-[(2S,3R,4S,5S,6R)-3,4,5-trihydroxy-6-(hydroxymethyl)oxan-2-yl] oxyphenyl]-1,3,3a,4,6,6a-hexahydrofuro[3,4-c]furan-3-yl]-2,6-dimethoxyphenoxy]-6-(hydroxymethyl)oxane-3,4,5-triol (eleutheroside E)
- 5,6,7,8-Tetrahydro-1,2,3,10,11,12-hexamethoxy-6,7-dimethyldibenzo[a,c]cycloocten-6-ol (schizandrin)
- 1,2,3,13-tetramethoxy-6,7-dimethyl-5,6,7,8-tetrahydrobenzo[3',4']cycloocta [1',2':4,5]benzo[1,2-d][1,3]dioxole (gamma- schizandrin)

The compounds are present in the composition as salts, solvates, isomers, hydrates, polymorphs or other modifications.

In a preferred embodiment the components of the composition are standardized with respect to the total amount of the composition for
- (2R,3S,4S,5R,6R)-2-(hydroxymethyl)-6-[2-(4-hydroxyphenyl)ethoxy]oxane-3,4,5-triol in an amount of about 0.01 to about 2.0 % w/w, preferably 0.05 to 1.5% w/w and more preferably 0.1 to 0.5 % w/w
- 4-(2-hydroxyethyl)phenol in an amount of about 0.01 to about 1.0 m % w/w, preferably 0.02 to 0.5% w/w and more preferably 0.04 to 0.1 % w/w
- 0.3% to about 0.5% 2-(3-phenylprop-2-enoxy)-6-[(3,4,5-trihydroxyoxan-2-yl)oxymethyl]oxane-3,4,5-triol in an amount of about 0.01 to about 3.0 % w/w, preferably 0.05 to 1.5% w/w and more preferably 0.1 to 0.5 % w/w
- (2R,3S,4S,5R,6S)-2-(hydroxymethyl)-6-[4-(3-hydroxyprop-1-enyl)-2,6-dimethoxyphenoxy]oxane-3,4,5-triol and (2R,3S,4R,5R,6S)-2-[4-[6-[3,5-dimethoxy-4-[(2S,3R,4S,5S,6R)-3,4,5-trihydroxy-6-(hydroxymethyl)oxan-2-yl] oxyphenyl]-1,3,3a,4,6,6a-hexahydrofuro[3,4-c]furan-3-yl]-2,6-dimethoxyphenoxy]-6-(hydroxymethyl)oxane-3,4,5-triol in an amount of about 0.005 to about 2.0 % w/w, preferably 0.008 to 1.0 % w/w and more preferably 0.01 to 0.2 % w/w
- 5,6,7,8-Tetrahydro-1,2,3,10,11,12-hexamethoxy-6,7-dimethyldibenzo[a,c]cycloocten-6-ol, and 1,2,3,13-tetramethoxy-6,7-dimethyl-5,6,7,8-tetrahydrobenzo[3',4']cycloocta [1',2':4,5]benzo[1,2-d][1,3]dioxole in an amount of about 0.01 to about 3.0 % w/w, preferably 0.03 to 1.5% w/w and more preferably 0.05 to 0.5 % w/w.
The composition further contains pantothenic acid or a salt thereof. Examples of a pantothenic acid salt are calcium, hemi calcium, sodium, potassium, ammonium pantothenate. This compound is present in an amount of 0.5 to 500 mg preferably 1 to 250 mg and more preferably 10 to 150 mg per single dose. In relation to the total amount of the composition the pantothenic acid or salt thereof is present in an amount of 1 to 50 %w/w and preferably 10 to 25 % w/w.

The extracts and the mixture of extracts are prepared by methods to achieve the presence of the compounds of the composition in the extract.

In a preferred embodiment the composition is a combination of extracts.

An extract may be prepared by the following method:
a) Extracting each plant material from the *Crassulaceae, Araliaceae* and *Schisandraceae* families by a hydro-alcoholic solvent. Typically, the solvent is an ethanol/water mixture ranging from 1% ethanol to 99% ethanol. Other alcohols, such as methanol and butanol may be used. Preferably, the extraction process is a specific validated process that meets the Good Manufacturing Practice standards of U.S. Food and Drug Administration. The temperature of the extraction procedure can be in a range between 20° C to 95° C depending on length of extraction time and quality of the raw material.
b) Separating the extraction solvent from the plant material.
c) Evaporating alcohol to obtain spissum.
d) Homogenizing each spissum which contains the combination of extracts.
e) Determination of concentration of marker compound in each spissum.
f) Mixing spissum and optionally pharmaceutically acceptable excipients in a ratio to achieve target amounts of marker compounds.
g) Evaporating spissum to dryness.
h) Determination of marker compounds in dry extract.
g) Adjusting concentration of marker compounds in dry extract by pharmaceutically acceptable excipient to achieve a defined amount of marker compound for the respective extracts in relation to the final amount of the composition:

After evaporating alcohol the extract contains a remaining amount of the extraction solvent. This extract is known as spissum.
The homogenization of the spissum is done by stirring or any other appropriate method. In a preferred embodiment the stirring is performed at an elevated temperature. Preferably the temperature for stirring is between 40 and 80 °C and more preferably between 50 and 70°C.

Examples for the drying steps are heating, spry drying or any other suitable methods.

Standardization of the parts of plants or extract is done by testing each extract by HPLC and TLC.
The extract of *Crassulaceae* is standardized to the content of salidroside, rosavin and/ or tyrosol.
The extract of *Araliaceae* is standardized to the content of eleutheroside B and/ or eleutheroside E.
The extract of *Schisandraceae* is standardized to the content of schisandrine and/ or gamma- schisandrine.

The dried extracts are further processed to manufacture pharmaceutical compositions or dietary food products. For this process pharmaceutically acceptable exipients may be used.
Examples of excipients are microcrystalline cellulose, cellulose derivatives, lactose, maltodextrines, talcum, gelatin, magnesium stearate, colloidal silicium, polyethylene glycoside and derivatives. Pharmaceutically acceptable antioxidants, preservatives, detergents, stabilizers may be present in the composition.

The final formulation of the compositions of the invention is any pharmaceutically acceptable formulation. Examples of formulations are a mixture of parts of plants, powder, solution, dispersion, suspension, granules, pellets, tablet, hard capsule, soft capsule, microcapsules, lozenge.

The formulation is applied once, twice, three or four times daily. A twice a day application is preferred. At each application time one, two, three, four or five, preferably two to four dosage units are applied.

### Reference Example 1

ADAPT-232 extract is prepared by extracting plant material from Eleutherococcus senticosus, Schizandra chinensis and Rhodiola rosea. The extraction is performed with 70% ethanol/water mixture for 6 hours at 60 °C. The liquid extract is separated from the plant material, concentrated by evaporation to spissum (water content about 40%).

The aliquot of the spissum are analyzed by HPLC and TLC and standardized for the certain content of analytical markers by blending of two soft extracts obtained from first and second extraction of the same raw material. Combined extract is homogenized and stabilized by addition of preservatives, mixed with certain amount of matodextrin, homogenized at room temperature for several hours and spray dried at elevated temperature of 75 to 200 °C.

The dry extracts are analyzed by TLC and HPLC and mixed in certain proportion to obtain homogenous powder standardized in relation to the total amount of composition for:
- (2R,3S,4S,5R,6R)-2-(hydroxymethyl)-6-[2-(4-hydroxyphenyl)ethoxy]oxane-3,4,5-triol 0.1 to 0.5 % w/w;
- (2R,3S,4S,5R,6S)-2-(hydroxymethyl)-6-[4-(3-hydroxyprop-1-enyl)-2,6-dimethoxyphenoxy] oxane-3,4,5-triol and (2R,3S,4R,5R,6S)-2-[4-[6-[3,5-dimethoxy-4-[(2S,3R,4S,5S,6R)-3,4,5-trihydroxy-6-(hydroxymethyl)oxan-2-yl]oxyphenyl]-1,3,3a,4,6,6a-hexahydrofuro[3,4-c]furan-3-yl]-2,6-dimethoxyphenoxy]-6-(hydroxymethyl)oxane-3,4,5-triol 0.01 to 0.2 % w/w;
- 5,6,7,8-Tetrahydro-1,2,3,10,11,12-hexamethoxy-6,7-dimethyldibenzo[a,c]cycloocten-6-ol, and 1,2,3,13-tetramethoxy-6,7-dimethyl-5,6,7,8-tetrahydrobenzo[3',4']cycloocta[1',2':4,5]benzo[1,2-d][1,3]dioxole 0.05 to 0.5 % w/w.

### Example 2

The extract is prepared according to reference example 1.

About 20% calcium pantothenate with respect to the composition is added.

### Reference Example 3

The amount of 380 mg of the dry extract of reference example 1 is mixed with 120 mg of excipients and filled in hard vegetable or gelatin capsules and packed into blisters.

In the final product the relative amount of (2R,3S,4S,5R,6R)-2-(hydroxymethyl)-6-[2-(4-hydroxyphenyl)ethoxy]oxane-3,4,5-triol is 0.16 %, the relative amount of (2R,3S,4S,5R,6S)-2-(hydroxymethyl)-6-[4-(3-hydroxyprop-1-enyl)-2,6-dimethoxyphenoxy]oxane-3,4,5-triol and (2R,3S,4R,5R,6S)-2-[4-[6-[3,5-dimethoxy-4-[(2S,3R,4S,5S,6R)-3,4,5-trihydroxy-6-(hydroxymethyl)oxan-2-yl]oxyphenyl]-1,3,3a,4,6,6a-hexahydrofuro[3,4-c]furan-3-yl]-2,6-dimethoxyphenoxy]-6-(hydroxymethyl)oxane-3,4,5-triol is 0.08 % and the amount of 5,6,7,8-Tetrahydro-1,2,3,10,11,12-hexamethoxy-6,7-dimethyldibenzo[a,c]cycloocten-6-ol, and 1,2,3,13-tetramethoxy-6,7-dimethyl-5,6,7,8-tetrahydrobenzo[3',4']cycloocta[1',2':4,5]benzo[1,2-d][1,3]dioxole is 0.25 %.

### Example 4

The amount of 440 mg of the dry extract of example 2 is mixed with 60 mg of excipients and filled in hard vegetable or gelatine capsules and packed into blisters.

In the final product the relative amount of 20% for calcium pantothenate.

### Reference Example 5

The individual extracts of reference example 1 and the combination of extracts of reference example 1 were dissolved in ethanol 0.8% and tested for deregulation of gene expression in neuroglia cell line. Human neuroglial cell line T98G (ATCC, CRL-1690) was grown in DMEM + GlutaMAX-I (Gibco, Darmstadt, Germany) with 10% foetal bovine serum (Gibco, Darmstadt) and 1% penicillin/streptomycin (Gibco, Darmstadt). Cells were maintained in a 37°C incubator in a humidified atmosphere with 5% CO2. All experiments were conducted using cells in the logarithmic growth phase. T98G cells were seeded 24 hours before treatment with extracts on 6-well plates in a density of 150,000 cells per well. The next day, old medium was removed and cells were treated in a final volume of 3 ml.

Two technical replicates were performed for each sample. Cells were incubated with the test substances for 24 hours at 37°C and then subjected to RNA isolation. Cells were harvested after 24 hours of treatment. Total RNA was isolated using InviTrap Spin Universal RNA Mini kit (Stratec Molecular, Berlin, Germany) and dissolved in RNAse free-water. The RNA of the two technical replicates was pooled (1:1) resulting in one sample for each treatment/control. The quality of total RNA was checked by gel analysis using the Total RNA Nano chip assay on an Agilent 2100 Bioanalyzer (Agilent Technologies GmbH, Berlin, Germany).

Microarray hybridizations were performed at the Institute of Molecular Biology (Mainz, Germany). Whole Human Genome RNA chips (8×60K Agilent) were used for gene expression profiling. Probe labelling and hybridization procedures were carried out following the One-Color Microarray-Based Gene Expression Analysis Protocol (http://www.chem.agilent.com/Library/usermanuals/Public/G4140900 40_GeneExpression_One-color_v6.5-pdf). Briefly, total RNA was labelled and converted to cDNA. Then, fluorescent cRNA (Cyanine 3-CTP) was synthesized and purified using the QIAgen RNeasy Kit. After fragmentation of the cRNA, samples were hybridized for 17 hours at 65°C. Microarray slides were washed and scanned with the Agilent Microarray Scanning system. Images were analyzed and data was extracted. The background was subtracted and data was normalized using the standard procedures of Agilent Feature Extraction Software. Expression data was further analyzed using Chipster software (http://chipster.csc.fi/) to filter genes by varying expression and significance. These steps include filtering genes to isolate those that were up- or down-regulated by one to three times the standard deviation (depending on the total number of extremely up- or down-regulated genes). A subsequent assessment of significance using empirical Bayes t-test further narrowed the pool of genes. All genes further considered showed a significant difference from the control with p-value < 0.05, or otherwise are noted. Filtered data was used in Ingenuity pathway analysis for Core analysis, in order to determine networks and pathways influenced by the drug treatments (http://www.ingenuity.com/).

A microarray-based transcriptome-wide mRNA expression analysis was performed to identify possible targets of the tested substances in T98G cells. T98G cells were treated with test substances for 24 h in two technical replicates before total RNA was isolated and pooled for microarray hybridization. Significantly deregulated genes were identified compared to untreated controls (p < 0.05) by means of Chipster software analysis.

The total number of deregulated genes in response to extracts was of the same order: 1075 - deregulated by Eleutherococcus, 1087 - deregulated by Schisandra, 1062 - deregulated by Rhodiola, and 1056 - deregulated by the combination ADAPT-232. Among the 1056 genes deregulated by ADAPT-232, there were 210 unique genes deregulated due to synergistic interaction of constituents (Table 1). Among them 89 genes are up regulated and 121 genes are down regulated more than two fold.

**Table 1: Genes up- and down-regulated by ADAPT- 232 combination of extracts in T98G cells. The values show fold changes compared to the control (http://www.ingenuity.com/wp-content/themes/ingenuity-qiagen/pdf/citation-guidelines/citing-ingenuity-products.pdf; Entrez Gene Name, http://www.ncbi.nlm.nih.gov/gene/).**

| Gene symbol - human (Entrez Gene) | Fold Change | Location | Type(s) |
|---|---|---|---|
| ZSCAN 10 | 18.765 | Nucleus | transcription regulator |
| PCDHAC1 | 8.754 | Plasma Membrane | other |
| HHAT | 8.574 | Cytoplasm | enzyme |
| FAM5B | 7.362 | Cytoplasm | other |
| PHACTR3 | 5.816 | Nucleus | other |
| B3GAT1 | 5.776 | Cytoplasm | enzyme |
| SMC1B | 5.657 | Nucleus | transporter |
| UBE3D | 5.278 | Other | other |
| SUCNR1 | 4.959 | Plasma Membrane | G-protein coupled receptor |
| C10rf105 | 4.925 | Other | other |
| OR2L13 | 4.627 | Plasma Membrane | G-protein coupled receptor |
| ANGPTL4 | 4.377 | Extracellular Space | other |
| OR4C3 | 4.317 | Plasma Membrane | G-protein coupled receptor |
| SRL | 4.199 | Cytoplasm | other |
| TEX26 | 4.199 | Other | other |
| SPTSSB | 4.084 | Cytoplasm | other |
| SLC16A10 | 3.864 | Plasma Membrane | transporter |
| TACR1 | 3.784 | Plasma Membrane | G-protein coupled receptor |
| SLC6A14 | 3.732 | Plasma Membrane | transporter |
| SYNPR | 3.655 | Plasma Membrane | transporter |
| TIFAB | 3.605 | Other | other |
| DTHD1 | 3.555 | Other | other |
| EPPIN | 3.555 | Extracellular Space | other |
| OR5T3 | 3.434 | Plasma Membrane | G-protein coupled receptor |
| PCDHGA8 | 3.434 | Other | other |
| LRRK2 | 3.340 | Cytoplasm | kinase |
| UBASH3A | 3.340 | Cytoplasm | enzyme |
| CCL18 | 3.272 | Extracellular Space | cytokine |
| ECHDC1 | 3.227 | Cytoplasm | enzyme |
| RPL28 | 3.227 | Cytoplasm | other |
| NKX2-4 | 3.182 | Nucleus | transcription regulator |
| KIF1A | 3.138 | Cytoplasm | other |
| TRBV2 | 3.138 | Other | other |
| C60rf222 | 3.095 | Other | other |
| ERP27 | 3.095 | Other | other |
| GRTP1 | 3.095 | Other | other |
| OR1A1 | 3.095 | Plasma Membrane | G-protein coupled receptor |
| RADIL | 3.095 | Other | other |
| ABCD2 | 3.074 | Cytoplasm | transporter |
| FAM107A | 3.053 | Nucleus | other |
| SRY | 3.053 | Nucleus | transcription regulator |
| DBX2 | 3.010 | Nucleus | transcription regulator |
| SPINK5 | 3.010 | Extracellular Space | other |
| SYT4 | 3.010 | Cytoplasm | transporter |
| BNIPL | 2.969 | Cytoplasm | other |
| CCKAR | 2.868 | Plasma Membrane | G-protein coupled receptor |
| OR51A4 | 2.828 | Plasma Membrane | other |
| CCDC173 | 2.789 | Other | other |
| SPESP1 | 2.751 | Cytoplasm | other |
| HM13 | 2.694 | Cytoplasm | peptidase |
| MAB21L1 | 2.694 | Nucleus | other |
| PCDH8 | 2.694 | Plasma Membrane | other |
| DLL4 | 2.585 | Extracellular Space | other |
| OSGIN1 | 2.567 | Other | growth factor |
| ANO6 | 2.549 | Plasma Membrane | ion channel |
| TEX36 | 2.549 | Other | other |
| C9orf57 | 2.532 | Other | other |
| TGM3 | 2.532 | Cytoplasm | enzyme |
| CCDC148 | 2.514 | Other | other |
| NOX5 | 2.497 | Cytoplasm | ion channel |
| C20orf160 | 2.479 | Other | other |
| PPP1R14D | 2.479 | Cytoplasm | other |
| DCDC1 | 2.462 | Other | other |
| HS3ST3B1 | 2.462 | Cytoplasm | enzyme |
| ANKS4B | 2.428 | Nucleus | transcription regulator |
| LILRA6 | 2.428 | Other | other |
| MMD2 | 2.428 | Other | other |
| RD3L | 2.428 | Other | other |
| SLCO4C1 | 2.428 | Plasma Membrane | transporter |
| LRP1B | 2.412 | Plasma Membrane | transmembrane receptor |
| OR2L2 | 2.412 | Plasma Membrane | G-protein coupled receptor |
| SERPINA4 | 2.412 | Extracellular Space | other |
| CHST9 | 2.395 | Cytoplasm | enzyme |
| LRRC19 | 2.395 | Other | other |
| RGS22 | 2.395 | Cytoplasm | other |
| THSD4 | 2.395 | Cytoplasm | other |
| ATXN8OS | 2.378 | Other | other |
| SPINK4 | 2.378 | Extracellular Space | other |
| C9orf64 | 2.362 | Other | other |
| LRFN5 | 2.362 | Nucleus | other |
| ZNF534 | 2.362 | Other | other |
| HPCAL1 | 2.346 | Cytoplasm | other |
| SHANK2 | 2.346 | Plasma Membrane | other |
| CXorf1 | 2.346 | Other | other |
| LDHAL6B | 2.329 | Cytoplasm | enzyme |
| MYH15 | 2.329 | Extracellular Space | other |
| NEUROD1 | 2.329 | Nucleus | transcription regulator |
| OR4D5 | 2.329 | Plasma Membrane | G-protein coupled receptor |
| LILRB1 | 2.313 | Plasma Membrane | transmembrane receptor |
| C1orf173 | -2.346 | Other | other |
| C2orf50 | -2.346 | Other | other |
| CD69 | -2.346 | Plasma Membrane | transmembrane receptor |
| CR1 | -2.346 | Plasma Membrane | transmembrane receptor |
| FAM179A | -2.346 | Other | other |
| HIGD1C | -2.346 | Other | other |
| IGSF1 | -2.346 | Plasma Membrane | other |
| LCE4A | -2.346 | Other | other |
| NETO1 | -2.346 | Extracellular Space | other |
| PCDH19 | -2.346 | Extracellular Space | other |
| RNASE9 | -2.346 | Extracellular Space | other |
| SLC35D3 | -2.346 | Other | other |
| ST8SIA6 | -2.346 | Cytoplasm | enzyme |
| ADAM20 | -2.362 | Plasma Membrane | peptidase |
| CTCFL | -2.362 | Nucleus | transcription regulator |
| GARNL3 | -2.362 | Other | other |
| OR5AC2 | -2.362 | Plasma Membrane | G-protein coupled receptor |
| SAMD3 | -2.362 | Other | other |
| WNT8B | -2.362 | Extracellular Space | other |
| SYNPO2 | -2.395 | Cytoplasm | other |
| PROZ | -2.428 | Extracellular Space | peptidase |
| ARMS2 | -2.445 | Cytoplasm | other |
| PCDHB18 | -2.445 | Other | other |
| RBP3 | -2.462 | Extracellular Space | transporter |
| SLC10A4 | -2.462 | Plasma Membrane | transporter |
| SLC17A6 | -2.462 | Plasma Membrane | transporter |
| PBLD | -2.479 | Other | enzyme |
| SPINT1 | -2.532 | Extracellular Space | other |
| CASQ1 | -2.549 | Cytoplasm | other |
| HCN1 | -2.549 | Plasma Membrane | ion channel |
| AGTR2 | -2.567 | Plasma Membrane | G-protein coupled receptor |
| SDPR | -2.567 | Plasma Membrane | other |
| SEPT14 | -2.567 | Cytoplasm | other |
| GOT1L1 | -2.585 | Other | enzyme |
| PTPRQ | -2.585 | Other | other |
| C12orf39 | -2.621 | Nucleus | other |
| C1orf100 | -2.621 | Other | other |
| LRTM2 | -2.621 | Other | other |
| OVCH1 | -2.621 | Other | other |
| PALMD | -2.621 | Cytoplasm | other |
| S100Z | -2.621 | Other | other |
| SH3BP5L | -2.621 | Other | other |
| SLC10A6 | -2.621 | Plasma Membrane | transporter |
| SLC18A1 | -2.621 | Plasma Membrane | transporter |
| CXorf59 | -2.639 | Other | other |
| IFNW1 | -2.676 | Extracellular Space | cytokine |
| IFNA8 | -2.751 | Extracellular Space | cytokine |
| SLC12A1 | -2.751 | Plasma Membrane | transporter |
| C3orf77 | -2.751 | Other | other |
| ZFP42 | -2.770 | Nucleus | transcription regulator |
| BMPER | -2.789 | Extracellular Space | other |
| FLJ40194 | -2.789 | Other | other |
| KRTAP10-11 | -2.789 | Other | other |
| OR4K15 | -2.789 | Plasma Membrane | G-protein coupled receptor |
| OR6C6 | -2.789 | Plasma Membrane | G-protein coupled receptor |
| SASH3 | -2.789 | Cytoplasm | other |
| SLC16A8 | -2.789 | Plasma Membrane | transporter |
| SPAG17 | -2.789 | Other | other |
| SPOCK3 | -2.789 | Extracellular Space | other |
| ZFP64 | -2.789 | Nucleus | other |
| COCH | -2.848 | Extracellular Space | other |
| FIGN | -2.868 | Nucleus | other |
| PPP1R9A | -2.868 | Plasma Membrane | other |
| TXLNB | -2.868 | Cytoplasm | other |
| TYR | -2.868 | Cytoplasm | enzyme |
| STAB1 | -2.928 | Plasma Membrane | transporter |
| C11orf16 | -2.949 | Other | other |
| GLP1R | -2.949 | Plasma Membrane | G-protein coupled receptor |
| ACMSD | -2.990 | Cytoplasm | enzyme |
| PCDHA1 | -2.990 | Plasma Membrane | other |
| CLEC5A | -3.010 | Plasma Membrane | other |
| OR4K5 | -3.010 | Plasma Membrane | G-protein coupled receptor |
| PCDHA6 | -3.010 | Plasma Membrane | other |
| RGR | -3.010 | Plasma Membrane | G-protein coupled receptor |
| SPO11 | -3.010 | Nucleus | enzyme |
| PCDH17 | -3.095 | Other | other |
| WIF1 | -3.095 | Extracellular Space | other |
| DNAJB3 | -3.117 | Other | other |
| IL7 | -3.117 | Extracellular Space | cytokine |
| SPTBN4 | -3.117 | Cytoplasm | other |
| TRAT1 | -3.117 | Plasma Membrane | kinase |
| RASSF9 | -3.204 | Cytoplasm | transporter |
| CTNNA3 | -3.294 | Plasma Membrane | other |
| FLT3 | -3.294 | Plasma Membrane | kinase |
| LRRIQ3 | -3.317 | Other | other |
| CCDC67 | -3.340 | Other | other |
| GC | -3.340 | Extracellular Space | transporter |
| TEKT5 | -3.340 | Other | other |
| UNC13C | -3.340 | Cytoplasm | other |
| ZNF396 | -3.340 | Nucleus | transcription regulator |
| GTSF1 | -3.411 | Cytoplasm | other |
| GH2 | -3.434 | Extracellular Space | other |
| TMPRSS11A | -3.434 | Other | peptidase |
| FAM65B | -3.506 | Other | other |
| SLC6A4 | -3.506 | Plasma Membrane | transporter |
| TRAV7 | -3.580 | Other | other |
| CDHR5 | -3.630 | Plasma Membrane | other |
| EPGN | -3.630 | Extracellular Space | growth factor |
| GUCY2F | -3.630 | Plasma Membrane | kinase |
| KIF12 | -3.630 | Cytoplasm | other |
| FPR2 | -3.681 | Plasma Membrane | G-protein coupled receptor |
| CCL22 | -3.732 | Extracellular Space | cytokine |
| FAM124A | -3-784 | Other | other |
| HEYL | -3-784 | Nucleus | transcription regulator |
| KRT75 | -3-784 | Cytoplasm | other |
| PTPN3 | -3.811 | Cytoplasm | phosphatase |
| BMP8B | -4.084 | Extracellular Space | growth factor |
| CYP26C1 | -4.141 | Cytoplasm | enzyme |
| TPD52L1 | -4.141 | Cytoplasm | other |
| TMPRSS11F | -4.438 | Other | peptidase |
| UNC45B | -4.959 | Cytoplasm | other |
| MAGEA8 | -5.169 | Other | other |
| C1orf61 | -5.618 | Cytoplasm | other |
| SAMSN1 | -6.277 | Nucleus | other |
| GPR151 | -6.869 | Plasma Membrane | G-protein coupled receptor |
| FAM153A | -7.434 | Other | other |
| KCNV1 | -7.835 | Plasma Membrane | ion channel |
| OR10G7 | -7.835 | Plasma Membrane | G-protein coupled receptor |
| EFCAB1 | -8.000 | Other | other |
| DYDC1 | -9.063 | Other | other |
| CD300LD | -9.448 | Plasma Membrane | other |

In the tables 2 to 15 below the genes affected by synergistic effect of the combination of extracts of example 1 in relation to the specific disorder are shown.

**Table 2: Synergy induced effect of ADAPT on genes involved in ageing related cardiovascular disorders.**

| **Disease or Function Annotation** | **p-Value** | **Entrez Gene N ame** |
|---|---|---|
| fibrosis of cardiac valve | 1.08E-02 | *SLC6A4* |
| fibrosis of valve leaflet | 1.08E-02 | *SLC6A4* |
| regression of artery | 1.08E-02 | *DLL4* |
| fibrosis of perivascular cuff | 2.14E-02 | *AGTR2* |
| congestive heart failure | 2.62E-02 | *GLP1R,SLC12A1,SLC6A4,STAB1* |
| activation of endothelial cells | 3.53E-03 | *BMPER,DLL4,FPR2,SERPINA4* |
| angiogenesis of capillary vessel | 9.66E-03 | *GH2,STAB1* |
| pH of vascular smooth muscle cells | 1.08E-02 | *AGTR2* |
| patterning of umbilical artery | 1.08E-02 | *DLL4* |
| remodeling of vasculature | 1.93E-02 | *DLL4,SLC6A4* |
| abnormal morphology of common cardinal vein | 2.14E-02 | *DLL4* |
| diastolic pressure | 2.25E-02 | *AGTR2,GLP1R,LRRK2* |
| abnormal morphology of atretic vasculature | 3.20E-02 | *DLL4* |
| development of capillary plexus | 3.20E-02 | *DLL4* |
| network formation of vascular endothelial cells | 3.20E-02 | *DLL4* |
| angiogenesis of leg | 4.24E-02 | *AGTR2* |
| cardiac contractility of left ventricle | 4.24E-02 | *GLP1R* |

**Table 3: Synergy induced effect of ADAPT on genes involved in ageing related cell death.**

| **Disease or Function Annotation** | **p-Value** | **Entrez Gene N ame** |
|---|---|---|
| cell death of leukemic blasts | 3.44E-04 | *FLT3,IL7* |
| apoptosis of T cell acute lymphoblastic leukemia cells | 1.08E-02 | *IL7* |
| apoptosis of induced pluripotent stem cells | 1.08E-02 | *LRRK2* |
| breakdown of oocytes | 1.08E-02 | *IL7* |
| degeneration of dorsal root ganglion cells | 1.08E-02 | *ABCD2* |
| delay in apoptosis of pre-B lymphocytes | 1.08E-02 | *IL7* |
| regeneration of granulocytes | 1.08E-02 | *IL7* |
| regeneration of megakaryocytes | 1.08E-02 | *IL7* |
| survival of lymphoid tissue-inducing cells | 1.08E-02 | *IL7* |
| survival of natural killer-22 cells | 1.08E-02 | *IL7* |
| survival of retinal rods | 1.08E-02 | *NEUROD1* |
| apoptosis of leukemic blasts | 2.14E-02 | *IL7* |
| cell viability of DN2 cells | 2.14E-02 | *IL7* |
| cell viability of pro T4 thymocytes | 2.14E-02 | *IL7* |
| cell viability of pro-T3 thymocytes | 2.14E-02 | *IL7* |
| survival of peripheral T lymphocyte | 2.14E-02 | *IL7* |
| apoptosis of medial smooth muscle cells | 3.20E-02 | *AGTR2* |
| apoptosis of recent thymic emigrants | 3.20E-02 | *IL7* |
| cell viability of TREG cells | 3.20E-02 | *IL7* |
| cellular degradation | 4.13E-02 | *ABCD2,IL7,KIF1A,LRRK2, PTPRQ* |
| survival of effector T lymphocytes | 4.24E-02 | *IL7* |
| survival of natural killer T lymphocytes | 4.24E-02 | *IL7* |

**Table 4: Synergy induced effect of ADAPT on genes involved in ageing related to auditory diseases and functions.**

| **Disease or Function Annotation** | **p-Value** | **Entrez Gene Name** |
|---|---|---|
| differentiation of cochlear duct | 1.08E-02 | *NEUROD1* |
| patterning of cochlear duct | 1.08E-02 | *NEUROD1* |
| orientation of stereocilia bundles | 1.75E-02 | *PTPRQ, WIF1* |
| fusion of stereocilia in inner hair cells | 2.14E-02 | *PTPRQ* |
| patterning of sensory epithelium | 2.14E-02 | *NEUROD1* |
| differentiation of sensory epithelium | 4.24E-02 | *NEUROD1* |
| autosomal recessive deafness type 84 | 1.08E-02 | *PTPRQ* |
| nonsyndromic deafness (DFNA9) | 3.20E-02 | *COCH* |

**Table 5: Synergy induced effect of ADAPT on genes involved in ageing related to cell degeneration.**

| **Disease or Function Annotation** | **p-Value** | **Entrez Gene Name** |
|---|---|---|
| degeneration of germ cells | 5.97E-03 | *BMP8B,SPO11* |
| degeneration of dorsal root ganglion cells | 1.08E-02 | *ABCD2* |
| degeneration of cells | 1. 72E-02 | *ABCD2,BMP8B,GUCY2F,KIF1A, LRRK2,PTPRQ,RBPg,SPO11* |
| injury of dopaminergic neurons | 2.14E-02 | *DLL4* |
| atrophy of acinar gland cells | 3.20E-02 | *AGTR2* |
| degeneration of neuroblastoma cell lines | 3.20E-02 | *LRRK2* |
| disruption of microvascular endothelial cells | 3.20E-02 | *ANGPTL4* |
| dysfunction of retinal cone cells | 3.20E-02 | *RBP3* |
| vacuolation of cardiomyocytes | 3.20E-02 | *AGTR2* |
| degeneration of male germ cells | 4.24E-02 | *BMP8B* |

**Table 6: Synergy induced effect of ADAPT on genes involved in ageing related to dermatological diseases.**

| **Disease or Function Annotation** | **p-Value** | **Entrez Gene Name** |
|---|---|---|
| malignant cutaneous melanoma cancer | 7.19E-03 | *BRINP2,C1orfl73,CCDC148,CHDC2, HCN1,LRP1B,NETOI,OR4D5,OR51A4, PCDHA1,PTPN3,SLCO4C1,SPOCK3, STAB1,TGM3,TYR* |
| focal facial dermal dysplasia type 4 | 1.08E-02 | *CYP26C1* |
| oculocutaneous albinism type 1B | 1.08E-02 | *TYR* |
| susceptibility to pseudofolliculitis barbae | 1.08E-02 | *KRT75* |
| tyrosinase-negative oculocutaneous albinism | 1.08E-02 | *TYR* |
| vitiligo vulgaris | 1.26E-02 | *TYR,UBASH3A* |
| skin cancer | 1.54E-02 | *ANGPTL4,BRINP2,C1orf173,CCDC148, CCL18,CHDC2,HCN1,LRP1B,NETO1, OR4D5,OR51A4,PCDHA1,PTPN3, SLCO4C1,SPOCK3,STAB1,TGM3, TYR* |
| melasma | 4.24E-02 | *TYR* |
| malignant cutaneous melanoma cancer | 7.19E-03 | *BRINP2,C1orf173,CCDC148,CHDC2,H CN1, LRP1B,NETO1,OR4D5,OR51A4,PCDH A1, PTPN3,SLCO4C1,SPOCK3,STAB1,TG M3,TYR* |
| focal facial dermal dysplasia type 4 | 1.08E-02 | *CYP26C1* |
| oculocutaneous albinism type 1B | 1.08E-02 | *TYR* |
| susceptibility to pseudofolliculitis barbae | 1.08E-02 | *KRT75* |
| tyrosinase-negative oculocutaneous albinism | 1.08E-02 | *TYR* |
| vitiligo vulgaris | 1.26E-02 | *TYR,UBASH3A* |
| skin cancer | 1.54E-02 | *ANGPTL4,BRINP2,C1orf173,CCDC148, CCL18,CHDC2,HCN1,LRP1B,NETO1, OR4D5,OR51A4,PCDHA1,PTPN3, SLCO4C1,SPOCK3,STAB1,TGM3,TYR* |
| melasma | 4.24E-02 | *TYR* |

**Table 7: Synergy induced effect of ADAPT on genes involved in ageing related digestive system disorders.**

| **Disease or Function Annotation** | **p-Value** | **Entrez Gene Name** |
|---|---|---|
| abnormal intestinal transit time of small intestine | 1.08E-02 | *CCKAR* |
| formation of cecal patch | 1.08E-02 | *IL7* |
| development of enteroendocrine cells | 2.14E-02 | *NEUROD1* |
| volume of gall bladder | 2.14E-02 | *CCKAR* |
| lack of islets of Langerhans | 3.20E-02 | *NEUROD1* |
| abnormal morphology of intestinal mucosa | 3.88E-02 | *ANGPTL4,NEUROD1* |

**Table 8: Synergy induced effect of ADAPT on genes involved in ageing related to endocrine system disorders.**

| **Disease or Function Annotation** | **p-Value** | **Entrez Gene Name** |
|---|---|---|
| abnormal secretion by pancreas | 3.10E-03 | *CCKAR,GLP1R* |
| Bartter syndrome type 1 | 1.08E-02 | *SLC12A1* |
| central hypothyroidism and testicular enlargement | 1.08E-02 | *IGSF1* |
| edema of pancreatic tissue | 1.08E-02 | *CR1* |
| maturity-onset diabetes of the young, type VI | 1.08E-02 | *NEUROD1* |
| susceptibility to Graves' disease type 3 | 1.08E-02 | GC |
| maturity-onset diabetes of the young | 1.93E-02 | *GLP1R,NEUROD1* |
| acinar cell adenoma | 2.14E-02 | *CCKAR* |
| hyperplasia of C-cells | 2.14E-02 | *GLP1R* |
| autoimmune pancreatitis | 2.52E-02 | *GC,PBLD* |

**Table 9: Synergy induced effect of ADAPT on genes involved in ageing related to energy production.**

| **Disease or Function Annotation** | **p-Value** | **Entrez Gene Name** |
|---|---|---|
| oxidation of 11-cis-retinal | 1.08E-02 | *RBP3* |
| oxidation of 11-cis-retinol | 1.08E-02 | *RBP3* |
| oxidation of L-dopa | 2.14E-02 | TYR |
| beta-oxidation of 22:6(n-3) fatty acids | 3.20E-02 | ABCD2 |

**Table 10: Synergy induced effect of ADAPT on genes involved in ageing related gastrointestinal disorders.**

| **Disease or Function Annotation** | **p-Value** | **Entrez Gene Name** |
|---|---|---|
| colon adenocarcinoma | 3.94E-04 | *ACMSD,AGTR2,ANGPTL4,BRINP2,C1orf173,CC DC173,CDHR5,CHDC2,CTCFL,CTNNA3,FAM12 4A,FAM179A,FLT3,GARNL3,GOT1L1,GPR151,G UCY2F,HM13,KIF1A,LCE4A,LILRA6,LRFN5,LR P1B,LRRIQ3,LRRK2,MYH15,NEUROD1,OR2L1 3,OR4K5,OR5AC2,OVCH1,PCDH8,PCDHGA8,PTPRQ,RBP3,RGS22,SAMD3,SAMSN1,SEPT14,S LC17A6,SLCO4C1,SMC1B,SPO11,SPTBN4,SRL,S T8SIA6,STAB1,TACR1,TEX26, TGM3, TMPRSS11 F,TOPAZ1,TYR, UNC13C, UNC45BZFP64* |
| abnormal secretion by pancreas | 3.10E-03 | *CCKAR,GLP1R* |
| pediatric inflammatory bowel disease | 3.10E-03 | *PHACTR3,TACR1* |
| colon cancer | 3.12E-03 | *ACMSD,AGTR2,ANGPTL4,BRINP2,C1orf173,CC DC173,CDHR5,CHDC2,CTCFL,CTNNA3,FAM12 4A,FAM179A,FLT3,GARNL3,GOT1L1,GPR151,G UCY2F,HM13,KIF1A,LCE4A,LILRA6,LRFN5,LR P1B,LRRLQ3,LRRK2,MYH15,NEUROD1,OR2L1 3,OR4K5,OR5AC2,OVCH1,PCDH8,PCDHGA8,P TPRQ,RBP3,RGS22,SAMD3,SAMSN1,SEPT14,S LC17A6,SLCO4C1,SMC1B,SPO11,SPTBN4,SRL,S T8SIA6,STAB1,TACR1,TEX26,TGM3,TMPRSS11 F,TOPAZ1,TPD52L1,TYR,UNC13C,UNC45B,ZFP* 64 |
| edema of pancreatic tissue | 1.08E-02 | *CR1* |
| maturity-onset diabetes of the young, type VI | 1.08E-02 | *NEUROD1* |
| maturity-onset diabetes of the young | 1.93E-02 | *GLP1R,NEUROD1* |
| colorectal cancer | 2.09E-02 | *ACMSD,4GTR2,4NGPTL4,BRINP2,C1orf173,CC DC173,CDHR5,CHDC2,CTCFL,CTNNA3,FAM12 4A,FAM179A,FLT3,GARNL3,GOT1L1,GPR151,G UCY2F,HM13,IFNW1,KIF1A,LCE4A,LILRA6,LR FN5,LRP1B,LRRIQ3,LRRK2,MAB21L1,MYH15, NEUROD1,OR2L13,OR4K5,OR5AC2,OVCH1,PC DH8,PCDHGA8,PTPRQ,RBP3,RGS22,SAMD3,S AMSN1,SEPT14,SLC17A6,SLC04Cl,SMCiB,SPO 11,SPTBN4,SRL,ST8SIA6,STAB1,TACR1, TEX26, TGM3,TMPRSS11F,TOPAZ1,TPD52L1,TYR,UNC 13C,UNC45B,ZFP64* |
| acinar cell adenoma | 2.14E-02 | *CCKAR* |
| autoimmune pancreatitis | 2.52E-02 | *GC,PBLD* |
| irritable bowel syndrome | 2.52E-02 | *CCKAR,SLC6A4* |
| Sjogren's syndrome | 3.04E-02 | *CCL18,CCL22,CD69,IL7* |

**Table 11: Synergy induced effect of ADAPT on genes involved in ageing related to modulation immune functions.**

| **Disease or Function Annotation** | **p-Value** | **Entrez Gene Name** |
|---|---|---|
| proliferation of pro-B lymphocytes | 1.75E-02 | *FLT3,IL7* |
| exit from cell cycle progression of pre-B lymphocytes | 2.14E-02 | *IL7* |
| development of pro-B lymphocytes | 2.95E-02 | *FLT3,IL7* |
| chemoattraction of B lymphocytes | 3.20E-02 | *CCL18* |
| induction of pre-B lymphocytes | 3.20E-02 | *IL7* |
| generation of B lymphocytes | 3.40E-02 | *CD69,IL7* |
| differentiation of pro-B lymphocytes | 4.13E-02 | *FLT3,IL7* |
| quantity of pre-B1 lymphocytes | 4.24E-02 | *IL7* |
| expansion of B lymphocytes | 4.38E-02 | *DLL4,IL7* |
| chemotaxis of regulatory T lymphocytes | 1.16E-04 | *CCL18,CCL22* |
| cell movement of regulatory T lymphocytes | 7.51E-04 | *CCL18,CCL22,STAB1* |
| activation of leukocytes | 2.55E-03 | *CCL22,CD3OOLD,CLEC 5A,CR1,DLL4,FLT3,FPR 2,GC,IFNA8,IFNWI,IL7, LILRB1,SAMSN1,SASH 3,SUCNRI,TACRI,TRAV 7,TYR* |
| activation of mononuclear leukocytes | 4.09E-03 | *CCL22,CLEC5A,DLL4,F LT3,IFNA8,IFNWI,IL7, LILRB1,SAMSN1,SASH 3,SUCNR1,TACR1,TRA V 7,TYR* |
| migration of regulatory T lymphocytes | 4.92E-03 | *CCL22,STAB1* |
| activation of lymphocytes | 6.81E-03 | *CCL22,DLL4,FLT3,IFN A8,IFNW1,IL7,LILRB1,S AMSN1,SASH3,SUCNR1, TACR1,TRAV7,TYR* |
| cell movement of naive T lymphocytes | 7.11E-03 | *CCL18,CCL22* |
| accumulation of lymphoid tissue-inducing cells | 1.08E-02 | *IL7* |
| attraction of Th2 cells | 1.08E-02 | *CCL22* |
| chemoattraction of leukocyte cell lines | 1.08E-02 | *CCL22* |
| chemoattraction of regulatory T lymphocytes | 1.08E-02 | *CCL18* |
| delay in initiation of activation of natural killer cells | 1.08E-02 | *FLT3* |
| recruitment of peritoneal macrophages | 1.08E-02 | *CCL22* |
| activation of macrophages | 1.16E-02 | *CCL22,CR1,GC,IFNA8,I FAW1,IL7* |
| activation of phagocytes | 1.39E-02 | *CCL22,CD3OOLD,CLEC 5A,CR1,FPR2,GC,IFNA8, IFNW1,IL7* |
| chemotaxis of microglia | 1.41E-02 | *FPR2,LRRK2* |
| activation of myeloid cells | 1.44E-02 | *CCL22,CLEC5A,CR1,FP R2,GC,IFNA8,IFNW1,IL 7* |
| activation of T lymphocytes | 1.46E-02 | *CCL22,DLL4,IFNA8,IF NW1,IL7,LILRB1,SUCN R1,TACR1,TRAV7,TYR* |
| activation of helper T lymphocytes | 1.58E-02 | *DLL4,IFNA8,IFATW1* |
| activation of memory T lymphocytes | 2.12E-02 | *IFNA8,IFNW1* |
| chemoattraction of lymphocytes | 2.12E-02 | *CCL18,CCL22* |
| recruitment of Th2 memory cells | 2.14E-02 | *CCL22* |
| relocalization of T lymphocytes | 2.14E-02 | *CD69* |
| trafficking of regulatory T lymphocytes | 2.14E-02 | *CCL22* |
| migration of monocyte-derived dendritic cells | 2.52E-02 | *CCL22,FPR2* |
| adhesion of immune cells | 2.94E-02 | *BMPER,CCL22,CD300L D,CD69,CR1,FAM65B,F PR2,IL7,STAB1* |
| cell movement of thymocytes | 2.95E-02 | *CCL22,CD69* |
| aggregation of PBMCs | 3.20E-02 | *IL7* |
| chemoattraction of B lymphocytes | 3.20E-02 | *CCL18* |
| chemoattraction of natural killer cells | 3.20E-02 | *CCL22* |
| activation of Th1 cells | 3.40E-02 | *IFNA8,IFNW1* |
| attraction of T lymphocytes | 3.40E-02 | *CCL18,CCL22* |
| activation of mucosal-associated invariant T lymphocytes | 4.24E-02 | *TRAV7* |
| susceptibility to Graves' disease type 3 | 1.08E-02 | *GC* |
| vitiligo vulgaris | 1.26E-02 | *TYR, UBASH3A* |
| polyarticular juvenile rheumatoid arthritis | 1.46E-02 | *CD69,CR1,FPR2,SLC6A 4* |
| infection of memory T lymphocytes | 2.14E-02 | *IL7* |
| lesioning of para-aortic lymph nodes | 2.14E-02 | *SPINT1* |
| autoimmune pancreatitis | 2.52E-02 | *GC,PBLD* |
| Sjogren's syndrome | 3.04E-02 | *CCL18,CCL22,CD69,IL7* |
| infection of naive T lymphocytes | 4.24E-02 | *IL7* |

**Table 12: Synergy induced effect of ADAPT on genes involved in ageing related to inflammatory response.**

| **Disease or Function Annotation** | **p-Value** | **Entrez Gene Name** |
|---|---|---|
| polyarticular juvenile rheumatoid arthritis | 1.46E-02 | *CD69,CR1,FPR2,SLC6A* 4 |
| autoimmune pancreatitis | 2.52E-02 | *GC,PBLD* |
| activation of leukocytes | 2.55E-03 | *CCL22,CD3OOLD,CLEC 5A,CR1,DLL4,FLT3,FPR 2,GC,IFNA8,IFNW1,IL7, LILRB1,SAMSN1,SASH 3,SUCNR1,TACR1,TRAV 7,TYR* |
| activation of mononuclear leukocytes | 4.09E-03 | *CCL22,CLEC5A,DLL4,F LT3,IFNA8,IFNW1,IL7, LILRB1,SAMSN1,SASH 3,SUCNR1,TACR1,TRAV 7,TYR* |
| proliferation of dendritic cells | 6.27E-03 | *FLT3,IFNA8,IFNW1* |
| activation of lymphocytes | 6.81E-03 | *CCL22,DLL4,FLT3,IFN A8,IFNW1,IL7,LILRB1,S AMSNI,SASH3,SUCNR1, TACR1,TRAV7,TYR* |
| accumulation of lymphoid tissue-inducing cells | 1.08E-02 | *IL7* |
| chemoattraction of regulatory T lymphocytes | 1.08E-02 | *CCL18* |
| delay in initiation of activation of natural killer cells | 1.08E-02 | *FLT3* |
| recruitment of peritoneal macrophages | 1.08E-02 | *CCL22* |
| activation of macrophages | 1.16E-02 | *CCL22,CR1,GC,IFNA8,I FAW1,IL7* |
| antimicrobial response | 1.25E-02 | *CCL22,CLECM,EPPIN, IFNA8,IFNW1,LILRB1,S TAB1* |
| activation of phagocytes | 1.39E-02 | *CCL22,CD3OOLD,CLEC 5A,CR1,FPR2,GC,IFNA8,IFNW1,IL7* |
| chemotaxis of microglia | 1.41E-02 | *FPR2,LRRK2* |
| activation of myeloid cells | 1.44E-02 | *CCL22,CLEC5A,CR1,FP R2,GC,IFNA8,IFNW1,IL 7* |
| activation of T lymphocytes | 1.46E-02 | *CCL22,DLL4,IFNA8,IF NW1,IL7,LILRB1,SUCN R1,TACR1,TRAV7,TYR* |
| activation of helper T lymphocytes | 1.58E-02 | *DLL4,IFNA8,IFNW1* |
| antiviral response | 1.65E-02 | *CCL22,CLEC5A,IFNA8, IFNW1,LILRB1* |
| activation of memory T lymphocytes | 2.12E-02 | *IFNA8,IFNW1* |
| chemoattraction of lymphocytes | 2.12E-02 | *CCL18,CCL22* |
| Th2 immune response of natural killer T lymphocytes | 2.14E-02 | *IL7* |
| expansion of bone marrow dendritic cell precursor | 2.14E-02 | *FLT3* |
| relocalization of T lymphocytes | 2.14E-02 | *CD69* |
| migration of monocyte-derived dendritic cells | 2.52E-02 | *CCL22,FPR2* |
| aggregation of PBMCs | 3.20E-02 | *IL7* |
| chemoattraction of B lymphocytes | 3.20E-02 | *CCL18* |
| chemoattraction of natural killer cells | 3.20E-02 | *CCL22* |
| inflammatory response of monocytes | 3.20E-02 | *CLEC5A* |
| activation of Th1 cells | 3.40E-02 | *IFNA8,IFNW1* |
| activation of mucosal-associated invariant T lymphocytes | 4.24E-02 | *TRAV7* |
| proliferation of dendritic precursor cells | 4.24E-02 | *FLT3* |
| inflammatory response | 4.66E-02 | *ABCD2,AGTR2,CCL18,C CL22,CD69,CLEC5A,CR 1,FPR2,GC,IFNA8,IFN W1,LL7,LRRK2,SASH3, TACR1* |

**Table 13: Synergy induced effect of ADAPT on genes involved in ageing related to lipid metabolism.**

| **Disease or Function Annotation** | **p-Value** | **Entrez Gene Name** |
|---|---|---|
| accumulation of very long chain fatty acid | 6.84E-04 | *ABCD2,CCL22* |
| agglomeration of cholesterol | 1.08E-02 | *CCKAR* |
| conversion of tretinoin | 1.08E-02 | *CYP26C1* |
| entrance of calcifediol | 1.08E-02 | *GC* |
| formation of 11-cis-retinal | 1.08E-02 | *RGR* |
| oxidation of 11-cis-retinal | 1.08E-02 | *RBP3* |
| oxidation of 11-cis-retinol | 1.08E-02 | *RBP3* |
| utilization of triacylglycerol | 1.08E-02 | *ANGPTL4* |
| dephosphorylation of phosphtidylinositol 5-phosphate | 2.14E-02 | *PTPRQ* |
| mobilization of all-trans-retinyl esters | 2.14E-02 | *RGR* |
| transport of vitamin D | 2.14E-02 | *GC* |
| beta-oxidation of 22:6(n-3) fatty acids | 3.20E-02 | *ABCD2* |
| dephosphorylation of phosphatidylinositol 3,5-diphosphate | 3.20E-02 | *PTPRQ* |
| dephosphorylation of phosphatidylinositol 4,5-diphosphate | 3.20E-02 | *PTPRQ* |
| dephosphorylation of phosphatidylinositol 4-phosphate | 3.20E-02 | *PTPRQ* |
| elongation of very long chain fatty acid | 3.20E-02 | *ABCD2* |
| concentration of corticosterone | 3.59E-02 | *AGTR2,GLP1R,SLC6A4, TACR1* |
| dephosphorylation of phosphatidylinositol 3,4-diphosphate | 4.24E-02 | *PTPRQ* |
| excretion of prostaglandin E2 | 4.24E-02 | *SLC12A1* |
| agglomeration of cholesterol | 1.08E-02 | *CCKAR* |

**Table 14: Synergy induced effect of ADAPT on genes involved in ageing related to metabolic diseases.**

| **Disease or Function Annotation** | **p-Value** | **Entrez Gene Name** |
|---|---|---|
| Bartter syndrome type 1 | 1.08E-02 | *SLC12A1* |
| central hypothyroidism and testicular enlargement | 1.08E-02 | *IGSF1* |
| deficiency of protein z | 1.08E-02 | *PROZ* |
| hypokalemic alkalosis | 1.08E-02 | *SLC12A1* |
| maturity-onset diabetes of the young, type VI | 1.08E-02 | *NEUROD1* |
| oculocutaneous albinism type 1B | 1.08E-02 | *TYR* |
| susceptibility to Graves' disease type 3 | 1.08E-02 | *GC* |
| susceptibility to reduced triglycerides | 1.08E-02 | *ANGPTL4* |
| tyrosinase-negative oculocutaneous albinism | 1.08E-02 | *TYR* |
| maturity-onset diabetes of the young | 1.93E-02 | *GLP1R,NEUROD1* |
| hypernatremia | 2.14E-02 | *SLC12A1* |
| ocular albinism with sensorineurial deafnes | 2.14E-02 | *TYR* |
| impaired fasting glucose | 3.20E-02 | *GLP1R* |

**Table 15: Synergy induced effect of ADAPT on genes involved in ageing related to cancer.**

| **Disease or Function Annotation** | **p-Value** | **Entrez Gene Name** |
|---|---|---|
| adenocarcinoma | 1.06E-05 | *ABCD2,ACMSD,ADAM20,AGTR2,ANGPTL4,B3GAT1,BNIPL, BRLNP2,C1orf173,CCDC173,CCKAR,CDHR5,CHDC2,CHST9, CR1,CTCFL,CTNNA3,DTHD1,FAM124A,FAM153A,FAM179A, FLT3,GARNL3,GC,GOT1L1,GPR151,GUCY2F,HM13,IGSF1,K IF1A,KRTAP10-11,LCE4A,LILRA6,LILRB1,LRFN5,LRP1B,LRRIQ3,LRRK2,M YH15,NEUROD1,OR10G7,OR2L13,OR2L2,OR4C3,OR4K5,O R5AC2,OR5T3,OVCH1,PCDH8,PCDHA6,PCDHB18,PCDHG A8,PTPRQ,RASSF9,RBP9,RGS22,SAMD3,SAMSN1,SASH3,S EPT14,SERPINA4,SLC16A10,SLC17A6,SLCO4C1,SMC1B,SPO 11,SPOCK3,SPTBN4,SRL,ST8SIA6,STAB1,SYNPO2,SYNPR,T ACR1,TEKT5,TEX26,TGM3,TIFAB,TMEM257,TMPRSS11F,T OPAZ1,TYR, UNC13C,UNC45B,WIF1,ZFP64,NF534* |
| colon adenocarcinoma | 3.94E-04 | *ACMSD,AGTR2,ANGPTL4,BRINP2,C1orf173,CCDC173,CDH R5,CHDC2,CTCFL,CTNNA3,FAM124A,FAM179A,FLT3,GAR NL3, GOT1L1,GPR151, GUCY2F,HM13,KIF1A,LCE4A,LILRA6, LRFN5,LRP1B,LRRIQ3,LRRK2,MYH15,NEUROD1,OR2L13, OR4K5,OR5AC2,OVCH1,PCDH8,PCDHGA8,PTPRQ,RBP3,R GS22,SAMD3,SAMSN1,SEPT14,SLC17A6,SLCO4C1,SMC1B,S PO11,SPTBN4,SRL,ST8SIA6,STAB1,TACR1,TEX26,TGM3,T MPRSS11F,TOPAZ1,TYR,UNC13C,UNC45B,ZFP64* |
| endometrioid carcinoma | 5.21E-04 | *AGTR2,BMPER,C1orf100,C9orf57,CASQ1,CCDC148,CCKAR, CR1,CTNNA3,DCDC1,DYDC1,FAM179A,FAM65B,FLT3,GUC Y2F,HCN1,IGSF1,KRT75,LRP1B,LRRC19,LRRIQ3,LRRK2,NETO1,NOX5,OR10G7,OR4D5,OR4K15,OR51A4,OR6C6,OVC H1,PCDH17,PCDH19,PCDHA6,PLSCR2,PPP1R9A,PTPN3,RA SSF9,RGS22,S100Z,SAMD3,SMC1B,SPINK5,SPOCK3,SPTSS B,STAB1,TACR1,TEKT5,TGM3,UNC13C,UNC45B,ZFP42,ZFP 64,ZNF396,ZNF534* |
| colon cancer | 3.12E-03 | *ACMSD,AGTR2,ANGPTL4,BRINP2,C1orf173,CCDC173,CDH R5,CHDC2,CTCFL,CTNNA3,FAM124A,FAM179A,FLT3,GAR NL3,GOT1L1,GPR151,GUCY2F,HM13,KIF1A,LCE4A,LILRA6, LRFN5,LRP1B,LRRIQ3,LRRK2,MYH15,NEUROD1,OR2L13, OR4K5,OR5AC2,OVCH1,PCDH8,PCDHGA8,PTPRQ,RBP3,R GS22,SAMD3,SAMSN1,SEPT14,SLC17A6,SLCO4C1,SMC1B,S PO11,SPTBN4,SRL,ST8SIA6,STAB1,TACR1,TEX26,TGM3,T MPRSS11F,TOPAZ1,TPD52L1,TYR,UNC13C,UNC45B,ZFP64* |
| malignant cutaneous melanoma cancer | 7.19E-03 | *BRINP2,C1orf173,CCDC148,CHDC2,HCN1,LRP1B,NETO1,O R4D5,OR51A4,PCDHA1,PTPN3,SLCO4C1,SPOCK3,STAB1,T GM3,TYR* |
| epithelial neoplasia | 9.17E-03 | *ABCD2,ACMSD,ADAM20,AGTR2,ANGPTL4,ANO6,B3GAT1, BNIPL,BRINP2,C1orf173,C1orf61,CCDC173,CCKAR,CDHR5, CHDC2,CHST9,CR1,CTCFL,CTNNA3,DTHD1,FAM124A,FA M153A,FAM179A,FLT3,FPR2,GARNL3,GC,GOT1L1,GPR151, GUCY2F,HCN1,HM13,IGSF1,KIF1A,KRTAP10-11,LCE4A,LILRA6,LILRB1,LRFN5,LRP1B,LRRIQ3,LRRK2,L RTM2,MYH15,NEUROD1,OR10G7,OR1A1,OR2L13,OR2L2,O R4C3,OR4K5,OR5AC2,OR5T3,OVCH1,PCDH19,PCDH8,PCD HA1,PCDHA6,PCDHB18,PCDHGA8,PTPRQ,RADIL,RASSF9, RBP3,RGS22,SAMD3,SAMSN1,SASH3,SEPT14,SERPINA4,S HANK2,SLC16A10,SLC17A6,SLCO4C1,SMC1B,SPAG17,SPIN K5,SPO11,SPOCK3,SPTBN4,SRL,ST8SIA6,STAB1,SYNPO2,S YNPR,TACR1,TEKT5,TEX26,TGM3,TIFAB,TMEM257,TMPR SS11F,TOPAZ1,TYR, UNC13C,UNC45B,WIF1,ZFP64,ZNF534* |
| carcinoma | 1.04E-02 | *ABCD2,ACMSD,ADAM20,AGTR2,4NGPTL4,B3GAT1,BNIPL ,BRINP2,C1orf173,C1orf61,CCDC173,CCKAR,CDHR5,CHDC2 ,CHST9,CR1,CTCFL,CTNNA3,DTHD1,FAM124A,FAM153A,F AM179A,FLT3,GARNL3,GC,GOT1L1,GPR151,GUCY2F,HCN1, HM13,IGSF1,KIF1A,KRTAP10-11,LCE4A,LILRA6,LILRB1,LRFN5,LRP1B,LRRIQ3,LRRK2,L RTM2,MYH15,NEUROD1,OR10G7,OR2L13,OR2L2,OR4C3,O R4K5,OR5AC2,OR5T3,OVCH1,PCDH19,PCDH8,PCDHA1,PC DHA6,PCDHB18,PCDHGA8,PTPRQ,RASSF9,RBP3,RGS22,SAMD3,SAMSN1,SASH3,SEPT14,SERPINA4,SHANK2,SLC16 A10,SLC17A6,SLCO4C1,SMC1B,SPAG17,SPINK5,SPO11,SPO CK3,SPTBN4,SRL,ST8SIA6,STAB1,SYNPO2,SYNPR,TACR1, TEKT5,TEX26,TGM3,TIFAB,TMEM257,TMPRSS11F,TOPAZ 1,TYR, UNC13C,UNC45B,WIF1,ZFP64,ZNF534* |
| expansion of leukemia cells | 1.08E-02 | *IL7* |
| metastasis of liver cell lines | 1.08E-02 | *C1orf61* |
| size of brain tumor | 1.08E-02 | *B3GAT1* |
| tumorigenesis of endothelial cells | 1.08E-02 | *ANGPTL4* |
| skin cancer | 1.54E-02 | *ANGPTL4,BRINP2,C1orf173,CCDC148,CCL18,CHDC2,HCN1, LRP1B,NETO1,OR4D5,OR51A4,PCDHA1,PTPN3,SLCO4C1,S POCK3,STAB1,TGM3,TYR* |
| colorectal cancer | 2.09E-02 | *ACMSD,AGTR2,ANGPTL4,BRINP2,C1orf173,CCDC173,CDH R5,CHDC2,CTCFL,CTNNA3,FAM124A,FAM179A,FLT3,GAR NL3,GOT1L1,GPR151,GUCY2F,HM13,IFNW1,KIF1A,LCE4A, LILRA6,LRFN5,LRP1B,LRRLQ3,LRRK2,MAB21L1,MYH15,N EUROD1,OR2L13,OR4K5,OR5AC2,OVCH1,PCDH8,PCDHGA 8,PTPRQ,RBP3,RGS22,SAMD3,SAMSN1,SEPT14,SLC17A6,S LCO4C1,SMC1B,SPO11,SPTBN4,SRL,ST8SIA6,STAB1,TACR1 ,TEX26,TGM3,TMPRSS11F,TOPAZ1,TPD52L1,TYR,UNC13C, UNC45B,ZFP64* |

### Example 6

The combination extract ADAPT-232 of reference example 1 and the combination extract ADAPT-232 with calcium pantothenate of example 2 were prepared in amylum suspension (1% w/v).

The randomised sets of experimental animals were divided into 4 study groups (control group C, ADAPT-232 groups Ai and A, and ADAPT-232 + D-panthenol group B) such that each group comprised one set of 5 male rats and one set of 5 female rats. The placebo and study drugs were administered intragastrically over a 4 month (120 day) period at a dose of 2 x 76 mg/kg ADAPT-232 per day with 10 h interval or 2 x 86 mg/kg ADAPT-232 with calcium pantothenate. In the Ai group treatment was interrupted after 30 days for a period of 14 days.

The study population consisted of 45 white outbreed male and female aged 2.0-2.1 years with weights in the range 380-420 g. Prior to selection, animals were submitted to a 14 day acclimatization period (quarantine). Animals were selected for inclusion in the study on the basis that their weight did not deviate by more than ± 5% from the population average for the gender. Animals were randomised into 9 sets of 5 animals (4 sets of male rats and 5 sets of female rats), and the members of each set were placed together in a cage, the label of which bore the identity numbers of the animals in that set.

Cages were maintained in separate rooms under a 12 h light - 12 h dark regime at an air temperature within the range 19-25°C and a relative humidity between 50 and 70%. The temperature and humidity were recorded daily whilst the levels of carbon dioxide and ammonia in the air were monitored constantly. The ventilation system employed was able to provide 15 facility volumes per hour, with carbon dioxide concentration not higher than 0.15 volume % and ammonia concentration not higher than 0.001 mg/l, at an air exchange rate controlled by an anemometer. The experimental animals were fed on standard granular fodder together with a mixed feed that included uncooked vegetables, bread, cottage cheese, vitamin food supplements and yeast. Rations were provided from a fodder trough fitted with a steel trellised cage cover appropriate for the age norm of the animals. Specially prepared filtered water was given ad libitum in standard autoclaved drinking bottles with steel tips.

At the end of the administration period animals were sacrificed.

### Example 6.1

The effect on the cells programmed death/ apoptosis was tested. Spleens were homogenised and filtered, and the erythrocytes destroyed by the addition of ammonium chloride solution. The lymphocytes were suspended in RPMI 1640 medium supplemented with gentamycin and 10% embryonic serum and the cell density determined by counting in a Gorjaev chamber. The lymphocyte suspension was fixed with 8% formalin solution and an equal volume of a 5 mg/ml solution of the DNA-specific fluorochrome Hoechst 33342 added. Samples were incubated at room temperature for 10 min, washed, and the ratio of cells with "normal" and "apoptotic" DNA in the nucleus estimated by fluorescent microscopy. To the positive control was added tumour necrosis factor-alpha (TNF-alpha) to a concentration of 500 U/ml, and actinomycin D to a concentration of 1 mg/ml. The level of apoptosis was determined as the percentage ratio of cells with nuclei containing condensed chromatin in comparison with cells containing diffused chromatin (100 cells in the visual field were counted).

The studied drugs reduced the level of apoptosis significantly with respect to the positive control, and the efficacy of the therapy applied was: B>A>Ai.

**Table 16: Effects of administration of the studied drugs for 120 days on the level of apoptosis in aged white rats.**

| Groups | Level of apoptosis (% ratio) | |
|---|---|---|
| | Males | Females |
| Native^{§} | 1.0 ± 0.1 | 1.1 ± 0.2* |
| Control (placebo) | 28.6 ± 3.3* | 31.1 ± 0.2* |
| Ai | 25.7 ± 3.6* | 23.5 ± 4.3* |
| A | 18.7 ± 3.3* | 18.1 ± 3.9* |
| B | 9.0 ± 0.7* | 9.0 ± 0.6* |
| Positive control^{†} | 80.0 ± 3.8 | 79.0 ± 5.0 |

| | | |
|---|---|---|
| The levels of apoptosis shown are arithmetic means ± SEM § Non-treated healthy 5 month old rats ^{∗} Indicates a significant difference of the mean value compared with that of the positive control group (P<0.05) † TNF-alpha stimulated splenocytes of aged rats | | |

### Example 6.2

The effect on the spontaneous occurrence of tumours was investigated. Each animal was examined and weighed every day throughout the drug administration period, and particular emphasis was given to the detection of tumours by palpation. Three of the experimental animals in the control group died in the middle of the 4th month of the study. The causes of death were: (i) pneumonia with hypostasis and leukocyte infiltration (in a male), (ii) suppurative inflammation of the uterine horns with abscesses and peritonitis (in a female), and (iii) unidentified virus infection with conjunctivitis and hemorrhagic alteration of the lungs and intestines (in a male).

**Table 17: Effects of administration of the studied drugs on the occurrence of tumours and the survival of aged white rats.**

| Groups | Gender | Group total before study | Days of administration of studied drug | | | | |
|---|---|---|---|---|---|---|---|
| | | | 30 days | 60 days | 90 days | 120 days | |
| | | | No. of deaths | No. of deaths | No. of deaths | No. diagnosed with tumours | No. of deaths |
| Control | Males | 5 | 0 | 0 | 0 | 2 | 2 |
| | Females | 5 | 0 | 0 | 0 | 1 | 1 |
| Ai | Males | 5 | 0 | 0 | 0 | 0 | 0 |
| | Females | 5 | 0 | 0 | 0 | 1 | 0 |
| A | Males | 5 | 0 | 0 | 0 | 0 | 0 |
| | Females | 5 | 0 | 0 | 0 | 1 | 0 |
| B | Males | 5 | 0 | 0 | 0 | 0 | 0 |
| | Females | 5 | 0 | 0 | 0 | 0 | 0 |

### Example 6.3

The effect on hypothalamus-pituitary-adrenal system activity and on lipid and protein metabolism was performed. In order to determine the levels of 17-oxycorticosteroids (17-OCS) on day 120 of the study period, urine was collected over a 24 h period by placing the animals in Tecniplast Gazzada metabolic cages. The assay of 17-OCS was based on the formation of a yellow coloured product when the analyte was heated with phenylhydrazine in the presence of sulphuric acid and ethanol. Since 17-OCS is mainly present in urine in the form of glucuronate and sulphate conjugates, the assay involved hydrolysis with glucoronidase followed by extraction with chloroform and dichloromethane, and subsequent addition of a mixture of sulphuric acid and ethanol. A control assay was carried out in order to determine the level of nonspecific colouration in the absence of phenylhydrazine.
At the end of the study period, the levels of 17-OCS, albumins and total proteins in the control animals had decreased from their baseline values of 11.3 ± 1.2 µmole/day, 49.1 ± 5.7 g/l and 75.5 ± 4.5 g/l, respectively. In contrast, the level of cholesterol increased over the study period from its baseline value of 1.36 ± 0.13 g/l, and this increase was particularly marked in male rats. Administration of ADAPT-232 + calcium pantothenate to male rats (group B) led to increased synthesis of 17-OCS, albumins and total proteins but decreased the level of cholesterol. In males of groups Ai and A, however, the only positive effect of the studied drugs was on total protein content. Similar results were observed for females in the respective groups with the exception that continuous administration of ADAPT-232 (group A) facilitated the decrease of cholesterol level.

The treatment B prevented age-specific dysfunctions of the hypothalamus - pituitary - adrenal system and decreased age-related hypercholesterolemia and hypoproteinemia. Treatment A was less effective, but it stabilized lipid-synthesis and prevented hypercholesterolemia in female rats.

**Table 18: Effects of administration of the studied drugs for 120 days on 17-oxycorticosteroids (17-OCS), cholesterol, lipid and protein metabolism in aged white rats.**

| Parameters | Groups | | | |
|---|---|---|---|---|
| | Control (placebo) | Ai | A | B |
| Males | | | | |
| 17-OCS (µmole/day) | 8.6 ± 0.6 | 9.6 ± 0.5 | 9.9 ± 0.4 | 11.1 ± 0.5* |
| Cholesterol (mmole/l) | 3.4 ± 0.3 | 2.8 ± 0.2 | 2.7 ± 0.2 | 2.3 ± 0.2* |
| Albumins (g/l) | 39.4 ± 1.0 | 42.9 ± 1.9 | 43.7 ± 2.0 | 45.3 ± 1.1* |
| Protein (g/l) | 69.8 ± 1.0 | 76.4 ± 2.0* | 76.8 ± 1.8* | 80.3 ± 1.2* |

| Females | | | | |
|---|---|---|---|---|
| 17-OCS (µmole/day) | 8.2 ± 0.5 | 9.1 ± 0.4 | 9.3 ± 0.5 | 10.4 ± 0.5* |
| Cholesterol (mmole/l) | 2.6 ± 0.1 | 2.2 ± 0.2 | 2.1 ± 0.2* | 1.8 ± 0.2* |
| Albumins (g/l) | 38.2 ± 1.0 | 41.3 ± 2.0 | 42.0 ± 2.0 | 43.5 ± 1.0* |
| Protein (g/l) | 69.2 ± 1.1 | 75.4 ± 2.1* | 76.8 ± 2.0* | 78.2 ± 2.0* |

| | | | | |
|---|---|---|---|---|
| Values shown are arithmetic means ± SEM * Indicates a significant difference of the mean value compared with that of the control group (P<0.05) | | | | |

### Example 7

The combination of extracts ADAPT-232 with calcium pantothenate of example 2 was diluted in 0.5% starch water and given orally at a dose of 67 mg/kg daily for 4 months (group 2). The combination of extracts ADAPT-232 with calcium pantothenate was given at a dose of 91 mg/kg (group 3). At the same time, the control group of aged rats received 0.5% starch water solution as placebo (group 1).

The male Wistar rats weighed 430-480 g and were 23-26 months old at the start of the experiment. Six months old male Wistar rats, weighing 340-380 g, were used as young controls. The animals were allowed at least 14 days to acclimatize.
The experimental groups included animals with no deviations in their appearance. After the acclimatisation period, the animals were randomly divided by body weight into 8 groups (ten rats per group); 2 control groups (aged rats and young adult rats) and 6 experimental groups (aged rats). The doses administered to each animal was based on their individual body weight.
The animals were fed a complete pellet diet which was available ad libitum. In addition, drinking water was also available ad libitum. Both food and water were available through a cave in the steel wire grid cover. The rats, five animals per cage, were kept in polycarbonate cages type 3H (Charles River Laboratories Inc) with a steel wire grid cover. Each cage was illuminated to give a cycle of 12 hours light and 12 hours darkness.

The room temperature was kept at about 20-26°C, with a relative humidity of 30% -70%, and the air exchange was controlled using an anemometer. Ammonium and CO₂ measurements were also taken. The ventilation system was designed to provide 15 air changes per hour to keep the CO₂ level at ≤ 0.15% v/v and the ammonium level at ≤ 0.001 mg/l.

### Example 7.1

The effects of the studied drugs on the blood cholesterol, HDL cholesterol and triglycerides of aged animals were evaluated. All animals were examined for blood biochemical parameters, such as total protein (TP), albumins, triglycerides (TRG), cholesterol and α-cholesterol (HDL) and.

Samples of fresh blood were collected from the inferior vena cava after overnight fasting and subsequent euthanasia (dead faint followed by cessation of breathing) of the experimental animals. Serum was isolated from the blood within 20 min of collection and the levels of lipids determined by automated colorimetric assay using a Cobas Integra (Roche, Hoffman La Roche Ltd, Basel, Switzerland) biochemical analyser and reagents. The method involved hydrolytic cleavage by cholesterol esterase of cholesterol and other sterol esters and triglycerides, followed by the action of peroxidase to yield a rose coloured product. The intensity of the absorption of the reaction mixture at 520 nm was linearly dependent on the cholesterol concentration, and the results were reported in mmol/l.

**Table 19: Blood biochemical parameters of aged rats assayed at day 0 (prior to administration) and day 120.**

| Group | N | Compo und | TRG (M ± SEM) mmol/l | LDH (M ± SEM) U\l |
|---|---|---|---|---|
| baseline | | | | |
| 1 | 10 | Placeb 0 | 0.49 ± 0.04 | 492.2 ± 29.3 |
| 2 | 10 | ADAPT | 0.58 ± 0.04 | 498.5 ± 13.6 |
| 3 | 10 | ADAPT | 0.52 ± 0.04 | 545.3 ± 30.5 |
| Day 120 | | | | |
| Young control | 10 | - | 0.29 ± 0.04 | 299.7 ± 11.2 |
| 1 | 7 | Placeb 0 | 0.54 ± 0.05■ | 507.0 ± 49.3■ |
| 2 | 8 | ADAPT | 0.64 ± 0.03■ | 421.8 ± 21.6■ |
| 3 | 9 | ADAPT | 0.53 ± 0.04■ | 284.5 ± 28.5• |

| | | | | |
|---|---|---|---|---|
| • - differences are statistically significant (p = 0.05) from the control group of aged animals (group 1) ■ - differences are statistically significant (p = 0.05) from the group of young adult animals | | | | |

### Example 7.2

The health status and mortality of rats during the experiment was investigated for the different treatment groups.

All animals showed normal appearance, behavioural reactions, motion and orientation activity at the start of the study and this was retained during administration of the test compounds. Indeed, there were no observed deviations in behaviour in all the groups of animals.

During the experiment cases of bronco-pulmonary diseases were observed in all groups of rats and the number of animals that fell ill and recovered from disease during administration of test compound were counted. The animals were considered ill when the following symptoms were observed: sneezing, coughing, complicated or infrequent breathing (accompanied with sound), discharges from nose and irritated mucosal membranes.

**Table 20: The number of rats that suffered from bronco-pulmonary diseases, percentage of mortality and recovery from diseases during a 4-month treatment.**

| Group | Compound | Animals fell ill, % | Animals recovered from disease, % | Animals demonstrated chronic disease | Animals died from disease, % | Total mortality, % |
|---|---|---|---|---|---|---|
| 1 | Placebo | 90 (9/10) | 11(1/9) | 55 (5/9) | 33 (3/9) | 33.3 (3/10) |
| 2 | ADAPT | 60 (6/10) | 50 (3/6) | 33 (2/6) | 16 (1/6) | 10 (1/10) |
| 3 | ADAPT | 50 (5/10) | 40 (2/5) | 40 (2/5) | 20 (1/5) | 10 (1/10) |

## Claims

1. Composition comprising a combination of the compounds (2R,3S,4S,5R,6R)-2-(hydroxymethyl)-6-[2-(4-hydroxyphenyl)ethoxy]oxane-3,4,5-triol, (2R,3S,4S,5R,6S)-2-(hydroxymethyl)-6-[4-(3-hydroxyprop-1-enyl)-2,6-dimethoxyphenoxy]oxane-3,4,5-triol, (2R,3S,4R,5R,6S)-2-[4-[6-[3,5-dimethoxy-4-[(2S,3R,4S,5S,6R)-3,4,5-trihydroxy-6-(hydroxymethyl)oxan-2-yl] oxyphenyl]-1,3,3a,4,6,6a-hexahydrofuro[3,4-c]furan-3-yl]-2,6-dimethoxyphenoxy]-6-(hydroxymethyl)oxane-3,4,5-triol, 5,6,7,8-Tetrahydro-1,2,3,10,11,12-hexamethoxy-6,7-dimethyldibenzo[a,c]cycloocten-6-ol, 1,2,3,13-tetramethoxy-6,7-dimethyl-5,6,7,8-tetrahydrobenzo[3',4']cycloocta [1',2':4,5]benzo[1,2-d] [1,3]dioxole,
pantothenic acid or a salt thereof,
and optionally pharmaceutically acceptable excipients for use in the prophylaxis, treatment and recovery of age related atherosclerosis.

2. Compositions for use according to claim 1 wherein the compounds of the composition act synergistically on the ZSCAN10, PCDHAC1, HHAT, FAM5B, PHACTR3, B3GAT1, SMC1B, UBE3D, SUCNR1, C10rf105, OR2L13, ANGPTL4, OR4C3, SRL, TEX26, SPTSSB, SLC16A10, TACR1, SLC6A14, SYNPR, TIFAB, DTHD1, EPPIN, OR5T3, PCDHGA8, LRRK2, UBASH3A, CCL18, ECHDC1, RPL28, NKX2-4, KIF1A, TRBV2, C6orf222, ERP27, GRTP1, OR1A1, RADIL, ABCD2, FAM107A, SRY, DBX2, SPINK5, SYT4, BNIPL, CCKAR, OR51A4, CCDC173, SPESP1, HM13, MAB21L1, PCDH8, DLL4, OSGIN1, ANO6, TEX36, C9orf57, TGM3, CCDC148, NOX5, C20orf160, PPP1R14D, DCDC1, HS3ST3B1, ANKS4B, LILRA6, MMD2, RD3L, SLCO4C1, LRP1B, OR2L2, SERPINA4, CHST9, LRRC19, RGS22, THSD4, ATXN8OS, SPINK4, C9orf64, LRFN5, ZNF534, HPCAL1, SHANK2, CXorf1, LDHAL6B, MYH15, NEUROD1, OR4D5, LILRB1, C1orf173, C2orf50, CD69, CR1, FAM179A, HIGD1C, IGSF1, LCE4A, NETO1, PCDH19, RNASE9, SLC35D3, ST8SIA6, ADAM20, CTCFL, GARNL3, OR5AC2, SAMD3, WNT8B, SYNPO2, PROZ, ARMS2, PCDHB18, RBP3, SLC10A4, SLC17A6, PBLD, SPINT1, CASQ1, HCN1, AGTR2, SDPR, SEPT14, GOT1L1, PTPRQ, C12orf39, C1orf100, LRTM2, OVCH1, PALMD, S100Z, SH3BP5L, SLC10A6, SLC18A1, CXorf59, IFNW1, IFNA8, SLC12A1, C3orf77, ZFP42, BMPER, FLJ40194, KRTAP10-11, OR4K15, OR6C6, SASH3, SLC16A8, SPAG17, SPOCK3, ZFP64, COCH, FIGN, PPP1R9A, TXLNB, TYR, STAB1, C11orf16, GLP1R, ACMSD, PCDHA1, CLEC5A, OR4K5, PCDHA6, RGR, SPO11, PCDH17, WIF1, DNAJB3, IL7, SPTBN4, TRAT1, RASSF9, CTNNA3, FLT3, LRRIQ3, CCDC67, GC, TEKT5, UNC13C, ZNF396, GTSF1, GH2, TMPRSS11A, FAM65B, SLC6A4, TRAV7, CDHR5, EPGN, GUCY2F, KIF12, FPR2, CCL22, FAM124A, HEYL, KRT75, PTPN3, BMP8B, CYP26C1, TPD52L1, TMPRSS11F, UNC45B, MAGEA8, C1orf61, SAMSN1, GPR151, FAM153A, KCNV1, OR10G7, EFCAB1, DYDC1 and/ or CD300LD.

3. Composition for use according to any of claims 1 to 2 wherein the composition comprises herbal material or extracts of plants belonging to the family of Crassulaceae, Araliaceae and Schisandraceae.

4. Composition for use according to any of claim 1 to 3 wherein the herbal material or extract is selected from the plants Sedum rosea, Schisandra chinensis and/or Eleutherococcus senticosus.

5. Composition for use according to any of claims 1 to 4 wherein (2R,3S,4S,5R,6R)-2-(hydroxymethyl)-6-[2-(4-hydroxyphenyl)ethoxy]oxane-3,4,5-triol is present in an amount of 0.01 to 2.0 % w/w, preferably 0.1 to 0.5 % w/w;
wherein (2R,3S,4S,5R,6S)-2-(hydroxymethyl)-6-[4-(3-hydroxyprop-1-enyl)-2,6-dimethoxyphenoxy]oxane-3,4,5-triol and (2R,3S,4R,5R,6S)-2-[4-[6-[3,5-dimethoxy-4-[(2S,3R,4S,5S,6R)-3,4,5-trihydroxy-6-(hydroxymethyl)oxan-2-yl] oxyphenyl]-1,3,3a,4,6,6a-hexahydrofuro[3,4-c]furan-3-yl]-2,6-dimethoxyphenoxy]-6-(hydroxymethyl)oxane-3,4,5-triol are present in an amount of 0.005 to 2.0 % w/w, preferably 0.01 to 0.2 % w/w;
wherein 5,6,7,8-Tetrahydro-1,2,3,10,11,12-hexamethoxy-6,7-dimethyldibenzo[a,c]cycloocten-6-ol, and 1,2,3,13-tetramethoxy-6,7-dimethyl-5,6,7,8-tetrahydrobenzo[3',4']cycloocta [1',2':4,5]benzo[1,2-d][1,3]dioxole - are present in an amount of 0.01 to 3.0 % w/w, preferably 0.05 to 0.5 % w/w.

6. Composition for use according to any of claims 1 to 5 wherein pantothenic acid or a salt thereof is present in an amount of 1 to 50 % w/w, preferably 10 to 25 % w/w.

7. Composition for use according to any of claims 1 to 6 wherein the composition is prepared to be applied 2 times daily in the amount of 2 capsules each.

## Patentansprüche

1. Zusammensetzung umfassend eine Kombination der Verbindungen (2R,3S,4S,5R,6R)-2-(Hydroxymethyl)-6-[2-(4-hydroxyphenyl)ethoxy]oxan-3,4,5-triol, (2R,3S,4S,5R,6S)-2-(Hydroxymethyl)-6-[4-(3-hydroxyprop-1-enyl)-2,6-dimethoxyphenoxy]oxan-3,4,5-triol, (2R,3S,4R,5R,6S)-2-[4-[6-[3,5-Dimethoxy-4-[(2S,3R,4S,5S,6R)-3,4,5-trihydroxy-6-(hydroxymethyl)oxan-2-yl] oxyphenyl]-1,3,3a,4,6,6a-hexahydrofuro[3,4-c]furan-3-yl]-2,6-dimethoxyphenoxy]-6-(hydroxymethyl)oxan-3,4,5-triol, 5,6,7,8-Tetrahydro-1,2,3,10,11,12-hexamethoxy-6,7-dimethyldibenzo[a,c]cycloocten-6-ol, 1,2,3,13-Tetramethoxy-6,7-dimethyl-5,6,7,8-tetrahydrobenzo[3',4']cycloocta [1',2':4,5]benzo[1,2-d][1,3]dioxol, Pantothensäure oder ein Salz davon,
und gegebenenfalls pharmazeutisch akzeptable Hilfsstoffe zur Verwendung in der Prophylaxe, Behandlung und Genesung von altersbedingter Atherosklerose.

2. Zusammensetzungen zur Verwendung nach Anspruch 1, wobei die Verbindungen der Zusammensetzung synergistisch auf ZSCAN10, PCDHAC1, HHAT, FAM5B, PHACTR3, B3GAT1, SMC1B, UBE3D, SUCNR1, C1orf105, OR2L13, ANGPTL4, OR4C3, SRL, TEX26, SPTSSB, SLC16A10, TACR1, SLC6A14, SYNPR, TIFAB, DTHD1, EPPIN, OR5T3, PCDHGA8, LRRK2, UBASH3A, CCL18, ECHDC1, RPL28, NKX2-4, KIF1A, TRBV2, C60rf222, ERP27, GRTP1, OR1A1, RADIL, ABCD2, FAM107A, SRY, DBX2, SPINK5, SYT4, BNIPL, CCKAR, OR51A4, CCDC173, SPESP1, HM13, MAB21L1, PCDH8, DLL4, OSGIN1, AN06, TEX36, C90rf57, TGM3, CCDC148, NOX5, C20orf160, PPP1R14D, DCDC1, HS3ST3B1, ANKS4B, LILRA6, MMD2, RD3L, SLCO4C1, LRP1B, OR2L2, SERPINA4, CHST9, LRRC19, RGS22, THSD4, ATXN8OS, SPINK4, C9orf64, LRFN5, ZNF534, HPCAL1, SHANK2, CXorf1, LDHAL6B, MYH15, NEUROD1, OR4D5, LILRB1, C1orf173, C2orf50, CD69, CR1, FAM179A, HIGD1C, IGSF1, LCE4A, NETO1, PCDH19, RNASE9, SLC35D3, ST8SIA6, ADAM20, CTCFL, GARNL3, OR5AC2, SAMD3, WNT8B, SYNPO2, PROZ, ARMS2, PCDHB18, RBP3, SLC10A4, SLC17A6, PBLD, SPINT1, CASQ1, HCN1, AGTR2, SDPR, SEPT14, GOT1L1, PTPRQ, C12orf39, C1orf100, LRTM2, OVCH1, PALMD, S100Z, SH3BP5L, SLC10A6, SLC18A1, CXorf59, IFNW1, IFNA8, SLC12A1, C3orf77, ZFP42, BMPER, FLJ40194, KRTAP10-11, OR4K15, OR6C6, SASH3, SLC16A8, SPAG17, SPOCK3, ZFP64, COCH, FIGN, PPP1R9A, TXLNB, TYR, STAB1, C11orf16, GLP1R, ACMSD, PCDHA1, CLEC5A, OR4K5, PCDHA6, RGR, SPO11, PCDH17, WIF1, DNAJB3, IL7, SPTBN4, TRAT1, RASSF9, CTNNA3, FLT3, LRRIQ3, CCDC67, GC, TEKT5, UNC13C, ZNF396, GTSF1, GH2, TMPRSS11A, FAM65B, SLC6A4, TRAV7, CDHR5, EPGN, GUCY2F, KIF12, FPR2, CCL22, FAM124A, HEYL, KRT75, PTPN3, BMP8B, CYP26C1, TPD52L1, TMPRSS11F, UNC45B, MAGEA8, C1orf61, SAMSN1, GPR151, FAM153A, KCNV1, OR10G7, EFCAB1, DYDC1 und/oder CD300LD wirken.

3. Zusammensetzung zur Verwendung nach einem der Ansprüche 1 bis 2, wobei die Zusammensetzung pflanzliches Material oder Extrakte von Pflanzen zugehörig zu der Familie der Crassulaceae, Araliaceae and Schisandraceae enthält.

4. Zusammensetzung zur Verwendung nach einem der Ansprüche 1 bis 3, wobei das pflanzliche Material oder Extrakt ausgewählt ist aus den Pflanzen Sedum rosea, Schisandra chinensis und/oder Eleutherococcus senticosus.

5. Zusammensetzung zur Verwendung nach einem der Ansprüche 1 bis 4, wobei (2R,3S,4S,5R,6R)-2-(Hydroxymethyl)-6-[2-(4-hydroxyphenyl)ethoxy]oxan-3,4,5-triol in einer Menge von 0,01 bis 2,0 % w/w enthalten ist, vorzugsweise 0,1 bis 0,5 % w/w; wobei (2R,3S,4S,5R,6S)-2-(Hydroxymethyl)-6-[4-(3-hydroxyprop-1-enyl)-2,6-dimethoxyphenoxy]oxan-3,4,5-triol und (2R,3S,4R,5R,6S)-2-[4-[6-[3,5-Dimethoxy-4-[(2S,3R,4S,5S,6R)-3,4,5-trihydroxy-6-(hydroxymethyl)oxan-2-yl] oxyphenyl]-1,3,3a,4,6,6a-hexahydrofuro[3,4-c]furan-3-yl]-2,6-dimethoxyphenoxy]-6-(hydroxymethyl)oxan-3,4,5-triol in einer Menge von 0,005 bis 2,0 % w/w, vorzugsweise 0,01 bis 0,2 % w/w vorhanden sind;
wobei 5,6,7,8-Tetrahydro-1,2,3,10,11,12-hexamethoxy-6,7-dimethyldibenzo[a,c]cycloocten-6-ol, und 1,2,3,13-Tetramethoxy-6,7-dimethyl-5,6,7,8-tetrahydrobenzo[3',4']cycloocta [1',2':4,5]benzo[1,2-d][1,3]dioxol in einer Menge von 0,01 bis 3,0 % w/w, vorzugsweise 0,05 bis 0,5 % w/w vorhanden sind.

6. Zusammensetzung zur Verwendung nach einem der Ansprüche 1 bis 5, wobei Pantothensäure oder ein Salz davon in einer Menge von 1 bis 50 % w/w, vorzugsweise 10 bis 25 % w/w vorhanden ist.

7. Zusammensetzung zur Verwendung nach einem der Ansprüche 1 bis 6, wobei die Zusammensetzung derart vorbereitet ist, zweimal täglich in einer Menge von jeweils zwei Kapseln eingenommen zu werden.

## Revendications

1. Composition comprenant une combinaison des composés (2R,3S,4S,5R,6R)-2-(hydroxyméthyl)-6-[2-(4-hydroxyphényl)éthoxy]oxane-3,4,5-triol, (2R,3S,4S,5R,6S)-2-hydroxyméthyl)-6-[4-(3-hydroxyprop-1-ényl)-2,6-diméthoxyphénolxy]oxane-3,4,5-triol, (2R,3S,4R,5R,6S)-2-[4-[6-[3,5-diméthoxy-4-[(2S,3R,4S,5S,6R)-3,4,5-trihydroxy-6-(hydroxyméthyl)oxan-2-yl]oxyphényl]-1,3,3a,4,6,6a-hexahydrofuro[3,4-c]furan-3-yl]-2,6-diméthoxyphénoxy]-6-(hydroxyméthyl)oxane-3,4,5-triol, 5,6,7,8-tétrahydro-1,2,3,10,11,12-hexaméthoxy-6,7-diméthyldibenzo[a,c]cyclooctén-6-ol, 1,2,3,13-tétraméthoxy-6,7-diméthyl-5,6,7,8-tétrahydrobenzo[3',4']cycloocta[1',2':4,5]benzo[1,2-d] [1, 3] dioxole,
l'acide pantothénique ou un sel de celui-ci,
et éventuellement des excipients pharmaceutiquement acceptables,
pour une utilisation pour la prophylaxie, le traitement et la guérison de l'athérosclérose liée à l'âge.

2. Composition pour une utilisation selon la revendication 1, dans laquelle les composés de la composition agissent d'une manière synergique sur ZSCAN10, PCDHAC1, HHAT, FAM5B, PHACTR3, B3GAT1, SMC1B, UBE3D, SUCNR1, C1orf105, OR2L13, ANGPTL4, OR4C3, SRL, TEX26, SPTSSB, SLC16A10, TACR1, SLC6A14, SYNPR, TIFAB, DTHD1, EPPIN, OR5T3, PCDHGA8, LRRK2, UBASH3A, CCL18, ECHDC1, RPL28, NKX2-4, KIF1A, TRBV2, C60rf222, ERP27, GRTP1, OR1A1, RADIL, ABCD2, FAM107A, SRY, DBX2, SPINK5, SYT4, BNIPL, CCKAR, OR51A4, CCDC173, SPESP1, HM13, MAD21L1, PCDH8, DLL4, OSGIN1, ANO6, TEX36, C90rf57, TGM3, CCDC148, NOX5, C20orf160, PPP1R14D, DCDC1, HS3ST3B1, ANKS4B, LILRA6, MMD2, RD3L, SLCO4C1, LRP1B, OR2L2, SERPINA4, CHST9, LRRC19, RGS22, THSD4, ATXN8OS, SPINK4, C9orf64, LRFN5, ZNF534, HPCAL1, SHANK2, CXorf1, LDHAL6B, MYH15, NEUROD1, OR4D5, LILRB1, C10orf173, C20orf50, CD69, CR1, FAM179A, HIGD1C, IGSF1, LCE4A, NETO1, PCDH19, RNASE9, SLC35D3, ST8SIA6, ADAM20, CTCFL, GARNL3, OR5AC2, SAMD3, WNT8B, SYNPO2, PROZ, ARMS2, PCDHB18, RBP3, SLC10A4, SLC17A6, PBLD, SPINT1, CASQ1, HCN1, AGTR2, SDPR, SEPT14, GOT1L1, PTPRQ, C12orf39, C10orf100, LRTM2, OVCH1, PALMD, S1007, SH3BP5L, SLC10A6, SLC18A1, CXorf59, IFNW1, IFNA8, SLC12A1, C3orf77, ZFP42, BMPER, FLJ40194, KRTAP10-11, OR4K15, OR6C6, SASH3, SLC16A8, SPAG17, SPOCK3, ZFP64, COCH, FIGN, PPP1R9A, TXLNB, TYR, STAB1, C11orf16, GLP1R, ACMSD, PCDHA1, CLEC5A, OR4K5, PCDHA6, RGR, SPO11, PCDH17, WIF1, DNAJB3, IL7, SPTBN4, TRAT1, RASSF9, CTNNA3, FLT3, LRRIQ3, CCDC67, GC, TEKT5, UNC13C, ZNF396, GTSF1, GH2, TMPRSS1A, FAM65B, SLC6A4, TRAV7, CDHR5, EPGN, GUCY2F, KIF12, FPR2, CCL22, FAM124A, HEYL, KRT75, PTPN3, BMP8B, CYP26C1, TPD52L1, TMPRSSS11F, UNC45B, MAGEA8, C1orf61, SAMSN1, GPR151, FAM153A, KCNV1, OR10G7, EFCAB1, DYDC1 et/ou CD300LD.

3. Composition pour une utilisation selon l'une quelconque des revendications 1 à 2, la composition comprenant un matériel de type herbe médicinale ou des extraits de plantes appartenant à la famille des Crassulaceae, Araliaceae et Schisandraceae.

4. Composition pour une utilisation selon l'une quelconque des revendications 1 à 3, dans laquelle la matériel de type herbe médicinale ou l'extrait est choisi parmi les plantes Sedum rosea, Schisandra chinensis et/ou Eleutherococcus senticosus.

5. Composition pour une utilisation selon l'une quelconque des revendications 1 à 4, dans laquelle le (2R,3S,4S,5R,6R)-2-(hydroxyméthyl)-6-[2-(4-hydroxyphényl)éthoxy]oxane-3,4,5-triol est présent en une quantité de 0,01 à 2,0 % p/p, de préférence de 0,1 à 0,5 % p/p ;
dans laquelle le 2R,3S,4S,5R,6S)-2-hydroxyméthyl)-6-[4-(3-hydroxyprop-1-ényl)-2,6-diméthoxyphénolxy]oxane-3,4,5-triol et le (2R,3S,4R,5R,6S)-2-[4-[6-[3,5-diméthoxy-4-[(2S,3R,4S,5S,6R)-3,4,5-trihydroxy-6-(hydroxyméthyl)oxan-2-yl]oxyphényl]-1,3,3a,4,6,6a-hexahydrofuro[3,4-c]furan-3-yl]-2,6-diméthoxyphénoxy]-6-(hydroxyméthyl)oxane-3,4,5-triol sont présents en une quantité de 0,005 à 2,0 % p/p, de préférence de 0,01 % à 0,2 % p/p ;
dans laquelle le 5,6,7,8-tétrahydro-1,2,3,10,11,12-hexaméthoxy-6,7-diméthyldibenzo[a,c]cyclooctén-6-ol et le 1,2,3,13-tétraméthoxy-6,7-diméthyl-5,6,7,8-tétrahydrobenzo[3',4']cycloocta[1',2':4,5]benzo[1,2-d] [1,3]dioxole sont présents en une quantité de 0,01 à 3,0 %, de préférence de 0,05 à 0,5 % p/p.

6. Composition pour une utilisation selon l'une quelconque des revendications 1 à 5, dans laquelle l'acide pantothénique est présent en une quantité de 1 à 50 % p/p, de préférence de 10 à 25 % p/p.

7. Composition pour une utilisation selon l'une quelconque des revendications 1 à 6 la composition étant préparée pour être appliquée 2 fois par jour, en une quantité de 2 gélules chaque fois.
